(19) Europäisches Patentamt
European Patent Office
Office européen des brevets

(11) **EP 1 889 039 B1**

(12) **EUROPÄISCHE PATENTSCHRIFT**

(45) Veröffentlichungstag und Bekanntmachung des Hinweises auf die Patenterteilung:
**22.04.2015   Patentblatt 2015/17**

(51) Int Cl.:
*A61B 5/00* (2006.01)     *G01N 21/17* (2006.01)
*G01N 21/47* (2006.01)     *G01N 21/64* (2006.01)

(21) Anmeldenummer: **06753223.4**

(22) Anmeldetag: **31.05.2006**

(86) Internationale Anmeldenummer:
**PCT/DE2006/000941**

(87) Internationale Veröffentlichungsnummer:
**WO 2006/128442 (07.12.2006 Gazette 2006/49)**

(54) **VERFAHREN UND VORRICHTUNG ZUR OPTISCHEN CHARAKTERISIERUNG VON GEWEBE**

METHOD AND APPARATUS FOR OPTICAL CHARACTERIZATION OF TISSUE

PROCEDE ET DISPOSITIF SERVANT A LA CARACTERISATION OPTIQUE DE TISSUS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **31.05.2005   DE 102005025243**
          **31.05.2005   DE 102005025241**
          **31.05.2005   DE 102005025242**

(43) Veröffentlichungstag der Anmeldung:
**20.02.2008   Patentblatt 2008/08**

(73) Patentinhaber: **W.O.M. World of Medicine AG**
**96333 Ludwigsstadt (DE)**

(72) Erfinder:
• **FREUDENBERG, Thomas**
  **12623 Berlin (DE)**

• **SCHÖNBORN, Karl-Heinz**
  **12355 Berlin (DE)**
• **Dr. PHILIPP, Carsten Michael**
  **D-11227 Berlin (DE)**

(74) Vertreter: **Baumgärtel, Gunnar**
**Patentanwälte Maikowski & Ninnemann**
**Postfach 15 09 20**
**10671 Berlin (DE)**

(56) Entgegenhaltungen:
**WO-A-00/07514          WO-A-02/28273**
**WO-A-97/11355          DE-A1- 19 927 724**
**US-A- 5 034 613          US-A- 5 413 108**
**US-A- 5 759 767          US-A1- 2002 122 246**
**US-A1- 2004 036 838**

**Beschreibung**

Die Erfindung betrifft ein Verfahren zur optischen Charakterisierung von aus Zellen gebildetem menschlichem oder tierischem Gewebe sowie eine Vorrichtung zur Durchführung des Verfahrens.

Die Diagnostik epidermaler, kutaner, adnexaler und mukosaler Veränderungen erfolgte bis zum Ende des 20. Jahrhunderts überwiegend klinisch. Seit den 80er Jahren haben sich durch die rasante Entwicklung der Technik, vor allem der Rechentechnik, eine Reihe bildgebender Verfahren in der Dermatologie etablieren können.

Von spezieller Relevanz ist dabei die Fluoreszenz-basierte Tumordiagnostik. Hier sind bereits mehrere Geräte auf dem Markt, die auf der linearen UV-Anregung mit einer Xenonlampe basieren. Ausgewertet wird in der Regel die Autofluoreszenzintensität in charakteristischen Wellenlängenbereichen (Storz, Richard-Wolf GmbH, Olympus) vor einem Weißlicht-Hintergrundbild.

Insbesondere wird von der Arbeitsgruppe H. van den Bergh untersucht, wie durch eine sequenziell mehrfarbige Anregung, Lebensdauerauflösung und spezielle Datenauswertung eine erhebliche Verbesserung der Diagnose erzielt werden kann [H. van den Bergh, Med. Laser Appl. 18 (2003)1, 20 und darin zitierte]. In allen Fällen liegt die laterale Auflösung im Sub-Millimeter-Bereich. Eine Differenzierung in der Tiefe ist aus prinzipiellen Gründen nicht möglich.

Dem Ziel, eine dreidimensionale Fluoreszenz-Darstellung des Gewebes mit akzeptablen Lichtausbeuten zu realisieren, kam man erst mit der Multiphotonen-Fluoreszenzmikroskopie näher (W. Denk, J.H. Strickler, W.W. Webb, "2-Photon laser scanning fluorescence microscopy," Science 248 (1990), 73-76). Bei diesem Verfahren wird die ortsaufgelöste Fluoreszenzdetektion der konfokalen Mikroskopie, die sehr geringe Fluoreszenzsignale und damit niedrige Abtastraten bedingt, durch die Lokalisierung mittels einer Multiphotonenanregung ersetzt. Außerdem können bei diesem Verfahren Wellenlängen im so genannten optischen Fenster des Gewebes (700nm - 1000nm) für die Anregung eingesetzt werden. Der Fokus der Forschungen auf diesem Gebiet liegt seit dem Beginn der Arbeiten auf der räumlich aufgelösten Darstellung der Konzentrationen verschiedener Biomoleküle im zellulären und subzellulären Bereich (Übersicht W.R. Zipfel et al Nature Biotechnology 21 (2003), 11, 1369-1377 und darin zitierte).

In das Feld der Multiphotonen-Mikroskopie (-Tomographie) ordnen sich auch die aktuellen Arbeiten der Gruppe von Prof. Karsten König ein (K. König et al J. Biomed. Opt. 8 (2003) 3, 432-439). Die mit einer Ortsauflösung im Sub-Mikrometerbereich dargestellten intrazellulären Strukturen erlauben Aussagen bezüglich Zellteilung und Stoffwechselverhalten einzelner Zellen und damit eine Therapiekontrolle, nicht jedoch die Untersuchung ausgedehnter Gewebsareale. Die diagnostischen Ansätze auf der Basis der Multiphotonenmikroskopie gehen von der Interpretation der intrazellulären Stoffwechselvorgänge und der zellulären Struktur aus.

Für die in relevante ortsaufgelöste Darstellung auf der Basis fokussierter Fluoreszenzanregung kann als Stand der Technik auf die seit langem etablierte konfokale Fluoreszenzmikroskopie verwiesen werden. Sie liefert horizontale Schnittbilder mit einer Auflösung im Bereich der verwendeten Wellenlänge. Die Vorteile der Zweiphotonenanregung bei diesem Verfahren, namentlich durch Anregung im 'optischen Fenster', sowie deutlich bessere Ortsauflösung auch in streuendem Gewebe wurden diskutiert und demonstriert [Review: W. R. Zipfel, R. M. Williams, W. W. Webb, nature biotechnology, 21/11, November 2003]. Beispielhaft seien hier die Arbeiten von Levene et al. [M. J. Levene et al. In vivo multiphoton microscopy of deep brain tissue. J Neurophysiol 91: 1908-1912, 2004] und Theer et al. [P. Theer et al., Two-Photon imaging to a depth of 1000 mm in living brain tissue... Optics Lett. VOL. 28(12), 2003] erwähnt, in denen Nervenzellen und Blutgefäße von Ratten und Mäusen in vivo in einer Tiefe von 1 mm dargestellt werden konnten. Diese Arbeiten belegen, dass konfokal nichtlinear angeregte Fluoreszenz auch aus großer Tiefe im Gewebe als ortsaufgelöste Information erfasst werden kann. Allerdings konnten in Bezug auf Tumordiagnostik mit diesen Verfahren bislang nur morphologische und interzelluläre Informationen und keine Aussagen über den Stoffwechselzustand von Gewebebereichen gewonnen werden. Die für die therapeutischen Entscheidungen wichtigen Informationen wie Ausbreitung und Gesamtaktivität eines Tumors konnten bislang nicht geliefert werden.

Mit den Schriften US5034613 und US6166385 und begleitenden Veröffentlichung wurde das Verfahren der Zwei-Photonen-Mikroskopie und später der Mehr-Photonen-Mikroskopie bekannt gemacht. Die Idee dieser Verfahren beruht darauf, dass die dreidimensionale Ortsauflösung durch die enge räumliche Begrenzung einer durch Zwei- oder Mehr-Photonen-Prozesse angeregten Fluoreszenz erreicht wird. Die Bildgewinnung selbst basiert auf der rasternden Abtastung des Untersuchungsgebietes mit dem Anregungsstrahlung.

Die Auslösung von Zwei- oder Mehrphotonen-Prozesse ist an die simultane Absorption von zwei oder mehr Photonen im Anregungsgebiet gebunden. Deshalb sind im Allgemeinen dafür hohe Intensitäten, wie sie gepulste Lasersysteme bereitstellen, erforderlich. Typischerweise sind dies, wenn man zur Vermeidung von Gewebeschädigungen im Anregungsgebiet die mittlere Leistung des Lasers begrenzen muss, Piko- oder Femtosekundenpulse bzw. Folgen von solchen Pulsen.

Die verfahrensimmanente Zwei- oder Mehr-Photonenabsorption bedingt auch eine entsprechende Intensitätsabhängigkeit des Prozesses: Bei einem Zwei-Photonenprozess ist die Ausbeute typischerweise quadratisch von der Intensität abhängig, bei einem Drei-Photonenprozess von der dritten Potenz der Intensität usw.

Die dreidimensionale Ortsauflösung des Verfahrens, für das hier beispielhaft die Zwei- und Mehr-Photonen-Mikroskopie angeführt werden, basiert auf zwei miteinander verknüpften Effekten, die anhand der typischerweise eingesetzten Lasersysteme beschrieben werden:

In der Bildebene der Optik, durch die die Anregungsstrahlung in das Targetmaterial fokussiert wird, ergibt sich die Begrenzung durch die Eigenschaften der Strahlquelle und der verwendeten Optik. Beschrieben wird dies durch den Radius der Strahltaille $w_0$, der sich für den häufigen Fall der Grundmode-Strahlung, auf die hier als Beispiel ohne Beschränkung der Allgemeinheit des Erfindungsgedankens Bezug genommen wird, wie folgt näherungsweise berechnet:

$$w_0 \approx \frac{2\lambda \cdot f}{\pi \cdot D}$$

Dabei ist $\lambda$ die Wellenlänge der Strahlung, f die Brennweite der verwendeten Optik und D der ausgeleuchtete Durchmesser bei Strahlungseintritt in die Optik. Wie man leicht erkennt, lässt sich der Durchmesser des Anregungsgebietes in der Bildebene der Optik beispielsweise durch Wahl des Verhältnisses f/D einstellen.

Entlang der Ausbreitungsrichtung der Strahlung (z-Richtung) nimmt der Radius w(z) des - hier wieder beispielhaft betrachteten - Grundmode-Bündels zu:

$$w(z) = w_0 \cdot \sqrt{1 + \left(\frac{z \cdot \lambda}{\pi \cdot w_0^2}\right)^2}$$

Mit der Zunahme des Radius des Bündels nimmt die Intensität der Anregungsstrahlung und damit die Ausbeute des Prozesses ab.

$$I(z) = \frac{I_0}{1 + \left(\frac{z \cdot \lambda}{\pi \cdot w_0^2}\right)^2} \qquad Rayleigh-Länge \quad z_0 = \frac{\pi \cdot w_0^2}{\lambda}$$

Dabei ist $I_0$ die Intensität im Fokus der Anregung. Charakterisiert wird dieser Tatbestand durch die sogenannte Rayleigh-Länge, die angibt, in welchem Abstand vom Fokus die Intensität auf die Hälfte abgefallen ist.

Entsprechend dem oben angeführten, quadratischen oder stärkeren Zusammenhang zwischen der Ausbeute des Prozesses und der Anregungsintensität wird deutlich, dass das Anregungsgebiet auch entlang der hier z-Achse genannten Ausbreitungsrichtung begrenzt ist.

Die Beschreibung dieser Zusammenhänge machen auch deutlich, dass es in dieser Anordnung nicht möglich ist, die Dimensionen des Anregungsgebietes unabhängig voneinander durch die Wahl der Anregungsstrahlung und der Optik festzulegen. Die Ausdehnung des Anregungsgebietes in z-Richtung wächst quadratisch mit dem Radius des Anregungsstrahles im Fokus.

Auf Grund dieser Tatsache gelingt es beispielsweise für die Multiphotonen-Mikroskopie nicht, Übersichtsmessungen mit verringerter Auflösung - also vergrößertem Fokus - und entsprechend erhöhter Ausbeute durchzuführen, ohne dass dadurch überproportional Auflösung in der Ausbreitungsrichtung der Anregungsstrahlung verloren ginge. Dieser Tatbestand ist insbesondere dann nachteilig, wenn zur Vermeidung unerwünschter Prozesse im Targetmaterial die Intensität der Anregungsstrahlung begrenzt ist.

Bei der gezielten nichtlinearer Anregung von Prozessen in organischen oder anorganischen Stoffen ist weiterhin Folgendes zu beachten:

Der Spot, in dem nichtlineare Prozesse ablaufen, ist als Taille eines Gaußstrahles geformt und bei nicht zu großen Aperturen vergleichsweise langgestreckt. Gewünscht für die Auswertung ist jedoch zumeist eine kompakte Form des Anregungsvolumens, d. h. eine, bei der der Durchmesser dieses Volumens und die Ausdehnung senkrecht zur Strahlsachse in etwa die gleiche Größenordnung haben.

Dabei wird grundsätzlich wie bei den genannten Methoden üblich das dreidimensionale Bild durch Scannen erzeugt. Durch zusätzliche, erfinderisch neue Methoden und apparative Einrichtungen, die im Folgenden offenbart sind, wird die im vorgenannten Absatz beschriebene Restriktion aufgehoben

Eine weitere, den Einsatz der nichtlinearen Fluoreszenzmethodiken außerhalb der klinischen und biologischen Forschung beschränkende Begrenzung der bisherigen Methodiken ist das geringe Volumen der Methoden gemäß dem Stand der Technik ist die geringe Ausdehnung des untersuchten Gewebsvolumens. Dies liegt in der Größenordnung von lateral 500 x 500 μm und in der Tiefe 200 bis 400 μm. Die Begrenzung ist einerseits wie bereits beschrieben durch die zu hohe Ortsauflösung begrenzt. Andererseits begrenzen auch die Datenmengen und -raten, die bei einer Echtzeitauswertung - wie sie für den Einsatz für die klinische Diagnostik und Früherkennung von Erkrankungen, insbesondere tumoröser Art notwendig ist - den höchst wünschenswerten klinischen Einsatz der Methodik.

Die gattungsbildende US 2002/122246 A1 offenbart ein Mikroskopiesystem mit einem Tastkopf, der ein Gitter und eine Linse zum Erzeugen von Licht mit mehreren spektralen Komponenten, die sich über einen Bereich eines zu untersuchenden Objektes erstrecken, umfasst.

Zudem offenbart die US-A-5 413 108 ein Verfahren zur Untersuchung eines zweidimensionalen Gebietes einer Gewebeprobe, wobei die Gewebeprobe mit Licht einer ersten und zweiten Wellenlänge bestrahlt und jeweils die Fluoreszenz ortsabhängig gemessen wird.

Darüber hinaus offenbart die WO 00/07514 A ein Verfahren zur Behandlung von pigmentiertem Gewebe durch Bestrahlung des Gewebes mit Licht, insbesondere unter Erzeugung einer Zwei-Photonen-Anregung.

Die DE 199 27 724 A1 beschreibt ein Zwei-Photonen-Endoskop oder Mikroskop mit einer optischen Faser zum Übertragen eines gepulsten Strahls über einen optischen Weg zwischen einer Strahlquelle und einem Gewebe und einer Dispergiereinrichtung, wobei in der optischen Faser auftretende Dispersion durch Dispersion umgekehrt wird, die durch die Dispergiereinrichtung erzeugt wird.

Der vorliegenden Erfindung liegt das Problem zugrunde, ein Verfahren und eine Vorrichtung zur optischen Charakterisierung von Gewebe anzugeben, das eine zuverlässigere Unterscheidung von gesundem und erkranktem Gewebe als bisher möglich erlaubt.

Die vorgenannte Problem wird durch das Verfahren mit den Merkmalen des Anspruchs 1 und durch die Vorrichtung gemäß dem Anspruch 28 gelöst. Besonders bevorzugte Ausführungsformen der Erfindung sind in den Unteransprüchen angegeben.

Danach wird ein Verfahren zur optischen Charakterisierung von aus Zellen gebildetem menschlichen oder tierischen Gewebe angegeben, das die Schritte aufweist:

- Bereitstellen einer Strahlquelle zum Aussenden gerichteter elektromagnetischer Strahlung;
- Bestrahlen des zu charakterisierenden Gewebes mit der Strahlung, wodurch im Gewebe eine für das Gewebe charakteristische Rückstrahlung erzeugt wird, wobei
- die in das Gewebe eingedrungene Strahlung innerhalb eines Anregungsbereiches eine ausreichende Intensität aufweist, um im Gewebe eine charakteristische Rückstrahlung anzuregen, wobei
- der von der Strahlquelle ausgesandten Strahlung ein derartiges Intensitätsprofil quer zu ihrer Ausbreitungsrichtung aufgeprägt wird, dass der Anregungsbereich eine Mehrzahl von Zellen des Gewebes überdeckt und die angeregte Rückstrahlung auf zellübergreifende Gewebeeigenschaften zurückgeht, wobei
- das Aufprägen des Intensitätsprofils dadurch erfolgt, dass die von der Strahlquelle ausgesandte Strahlung in mindestens zwei Teilstrahlen aufgeteilt wird, wobei der Anregungsbereich durch die Lage der Teilstrahlen festgelegt ist, und wobei
- die Teilstrahlen jeweils so in das Gewebe fokussiert werden, dass die Fokuspunkte für die Teilstrahlen beabstandet zueinander sind, wobei
- die Rückstrahlung summarisch aus den Fokalbereichen aller Teilstrahlen erfasst wird.

Das erfindungsgemäße Verfahren legt die Grundlage für ein innovatives Fluoreszenzdiagnostisches, bildgebendes, nicht-invasives Tumordiagnoseverfahren, das zur Früherkennung und zum Screening (in vivo und in vitro) geeignet ist. Das Verfahren liefert die Charakterisierung des Gewebe- bzw. Stoffwechselzustandes und damit von krankhaften, insbesondere tumorösen Veränderungen mit einer an der klinischen Zielstellung orientierten Aussagekraft und Ortsauflösung, die dem Arzt derzeit nicht zur Verfügung steht. Der Gewebezustand wird aus den spektroskopischen Daten in suprazellulärer Mittelung bestimmt und dem Arzt in einem mehrere Millimeter umfassenden morphologischen Schnittbild dargestellt, das eine Bewertung von Tumoren hinsichtlich ihrer Ausdehnung, Position und Aggressivität erlaubt.

Dem Patienten nutzen vor allem die verbesserte Früherkennung und Diagnostik. Durch diese können operative Eingriffe vermieden oder - im Falle der Lokalisierung von Frühstadien - durch minimal-invasive Methoden deutlich reduziert werden. Die Krankenversicherung spart Behandlungskosten durch im Vergleich zu den bisher unvermeidlichen Biopsien um zwei Drittel verringerte Kosten sowie durch deutliche Reduktion von Zahl und Schwere der operativen Eingriffe. Soweit chirurgische Eingriffe nicht vermieden oder durch minimal-invasive Methoden, wie PDT (Photo-dynamische Therapie) ersetzt werden können, werden die Schwere des Eingriffs und seine heilungsbezogenen und ästhetischen Auswirkungen deutlich verringert, weil die Abgrenzung zwischen gesundem und krankem Gewebe bedeutend exakter erfolgen kann als bisher.

Die neuartige Methode lässt sich aber auch für andere Mess- und Charaterisierungsaufgaben z. B. im Bereich der Pathologie, Biochemie, Biotechnologie, für lebende oder nicht vitale organische sowie für anorganische Stoffe in festem oder flüssigem Zustand vorteilhaft einsetzen. Der besseren Verständlichkeit halber sind im Folgenden alle Spezifika am Beispiel der Fluoreszenzdiagnose von Tumoren dargestellt, ohne damit die Allgemeingültigkeit und Breite des Erfindungsgedankens einschränken zu wollen.

Neben dem zunächst dargestellten Einsatzgebiet im Bereich der Haut sind weitere Anwendungsbereiche im Bereich der zugänglichen Körperhöhlen (HNO, Gynäkologie) und im endoskopisch zugänglichen Körperinneren (Gastro-Entereologie, Urologie, Bronchoskopie) Teil des Anwendungsgebietes der Erfindung.

In der Behandlung bösartiger Erkrankungen ist eine frühzeitige Erkennung, neben den Krebs-Frühstadien, vor allem der Vorstufen, für die Aussichten auf Heilung von ganz entscheidender Bedeutung. Zudem steigt durch die zunehmende Lebenserwartung die Inzi denz solcher Krebs-Vorstufen, was durch die Lebensgewohnheiten, wie z.B. intensive Sonnenbestrahlung, Erkrankungen durch sexuell übertragbare Viren und Umweltnoxen noch verstärkt wird. Als Beispiele seien hier genannt:

a) die aktinische Keratose, eine durch additive UV-Exposition ausgelöste Erkrankung, die in einen Hautkrebs, das Plattenepithelkarzinom übergehen kann und

b) die durch HPV-Viren ausgelösten genitalen Feigwarzen, "Condylome", die nicht nur bei Frauen für die Auslösung des Gebärmutterhalskrebs verantwortlich sind, sondern auch bei Männern und Frauen Krebse der Anogenital-Region induzieren können.

Während eine manifeste Krebserkrankung häufig eindeutig erkannt werden kann ("Blickdiagnose" in ca. 50 % der Erkrankungen), sind deren Vorstufen und ganz frühen Stadien bei der visuellen Diagnose häufig unauffällig. Dies ist ein Grund dafür, weshalb Screening-Untersuchungen nicht den gewünschten Effekt erbringen. Sie können entweder "falsch negativ" sein, also eine vorhandene Krebserkrankung nicht rechtzeitig erkennen und damit die Chancen einer Heilung durch minimal-invasive Behandlungen verpassen. Ebenso ungünstig sind "falsch positive" Diagnosen, welche neben der erheblichen psychischen Belastung der Patienten bis zum Ausschluss einer Krebsdiagnose auch viele aufwendige Untersuchungen nach sich ziehen, die nicht nur die Patienten zusätzlich belasten und gefährden, sondern auch erhebliche Folgekosten verursachen.

Andererseits gewinnen in der Behandlung epithelialer Tumoren minimal-invasive Verfahren wie die Cryotherapie sowie die Photodynamische Therapie und neuerdings auch die medikamentöse Behandlung (Wirkstoff Imiquimod) eine immer stärkere Bedeutung. Da diese Behandlungsverfahren aber ohne Herausschneiden ("Exzision") arbeiten, fehlt ihnen die Möglichkeit der histologischen Untersuchung, mit der nicht nur die Diagnose gesichert wird, sondern auch Informationen über die Aggressivität des Tumors ("Grading"), und über das Überschreiten der Grenzstrukturen und das Eindringen in das benachbarte Gewebe ("Staging") erhalten werden. Voraussetzung für ein Screening sind deshalb neben der eindeutigen Diagnosestellung durch die räumliche Zuordnung der Fluoreszenz auch eine Aussage über die Ausbreitung und den Invasionsgrad.

Daraus ergibt sich, dass erfindungsgemäße Verfahren die medizinisch-diagnostischen Verfahrensanforderungen nach einer ortsaufgelösten Diagnose eingebettet in den morphologischen Rahmen des Untersuchungsgebietes vorteilhaft erfüllt, dessen Dimensio nen entsprechend den klinischen Erfahrungen 4 mm lateral und 0,5 mm in der Tiefe nicht unterschreiten sollten.

In einer bevorzugten Weiterbildung des Verfahrens besteht das Aufprägen des Intensitätsprofils darin, die Strahlung über ein Objektiv in das Gewebe abzubilden. Dabei besitzt das Objektiv eine numerische Apertur von näherungsweise 0,3 bis 1,5.

Der Grundgedanke der Erfindung besteht darin, dass aus dem von der Strahlquelle generierten Anregungsstrahl mehrere, sich in ihrer Richtung unterscheidende Teilstrahlen geformt werden. Mit einer Fokussierungsoptik entsteht in deren Fokusebene entsprechend der Anzahl und Anordnung der Teilstrahlen ein Bündel von Foki. Wird deren Abstand in der Fokalebene geeignet eingestellt, so ergibt sich entlang der Ausbreitungsrichtung der Anregungsstrahlung eine von der Zahl der Teilstrahlen zumindest annähernd unabhängige Begrenzung des Mehrphotonen-Anregungsgebietes.

Besonders vorteilhaft ist, wenn die Rückstrahlung ein Fluoreszenzsignal, z.B. eine Zwei- oder Mehr-Photonen-Fluoreszenz ist. Die bisherigen Verfahren der klinischen Fluoreszenzdiagnostik, sowohl der Auto- als auch der Xenofluoreszenz, erfassen nur ein Summenbild oberflächlicher Gewebsschichten. Die Anregung erfolgt mit kurzwelligem UV- bzw. Blaulicht, wobei durch Auslöschung durch darüber liegendes nichtstoffwechselaktives Nekrosegewebe ein "falsch negativer" Befund erhoben werden kann, oder bei Tumorausbreitung unter die gesunde Haut die tatsächliche Ausbreitung und damit die Bestimmung der Tumorgröße falsch eingeschätzt wird. Diese Nachteile lassen sich durch enge räumliche Begrenzung einer NIR-Mehrphotonenanregung mit Lasern überwinden.

Die in den letzten Jahren entwickelten Verfahren der Multiphotonen-Mikroskopie nutzen diesen Weg der Anregung von Fluophoren im Gewebe. Sie zielen auf hohe Ortsauflösungen (<1 $\mu$m) zur Untersuchung zellbiologischer Vorgänge in subzellulärer Dimension ab. Die dafür eingesetzten Methoden der scannenden Mikroskopie limitieren das Abtastvolumen auf Gesichtsfeldabmessungen und Scantiefen in der Größenordnung von einigen hundert Mikrometern. Diese Werte lassen sich - anders als beim erfindungsgemäßen Verfahren - prinzipbedingt nicht auf die für den klinischen Einsatz geforderten oben genannten Dimensionen des Scanvolumens ausdehnen.

Darüber hinaus erlaubt die subzelluläre Auflösung der konventionellen Verfahrenanders als bei der suprazellulären Auflösung des erfindungsgemäßen Verfahrens - nicht die Ermittlung der Korrelation zwischen den Fluoreszenzspektren endogener Fluophore und den pathologischen Zuständen entsprechender Zellbereiche. Die Multiphotonen-Mikroskopie ist zu einem wirkungsvollen Instrument der zellbiologischen Forschung gereift. Der klinische Einsatz zum Tomorstaging ist nicht ihr eigentliches Einsatzgebiet.

Das Verfahren dient im Bereich der Tumordiagnostik dem Zweck, die Zahl der tatsächlich erforderlichen operativen Eingriffe drastisch zu verringern. Zuvorderst sind Hauttumore im Fokus der Behandlung, da diese ohne weiteres von außen zugänglich sind.Weitergehender, z. T. noch bedeutenderer Anwendungen ergeben sich intra- und extrakorporal durch endoskopische und intraoperative Applikatoreinrichtung mit integrierter Scanfunktion.

Bei entsprechender erfindungsgemäß vorgesehener Erweiterung und Miniaturisierung liegen auch in der endoskopische Diagnostik, vor allem für die Gynäkologie und HNOHeilkunde, weitreichende Zukunfts-Potenziale für die Erfindung. Insbesondere die Differentialdiagnose der Gebärmutterhalserkrankungen und sonstiger durch humane Papillomaviren induzierten Erkrankungen, aber auch oraler Leukoplakien und der lichenoiden Krebsvorstufen sind sehr ernstzunehmende Anwendungsgebiete der Erfindung.

Für das Tumorstaging und damit die Indikationsstellung zu minimal-invasiven Verfahren ist neben der Tumorgröße die Ausbreitungstiefe und die Stromainvasion von viel entscheidenderer Bedeutung. Die bisherigen Schnittbildverfahren, wie MRT, sind von ihrer räumlichen Auflösung nicht dazu in der Lage. Die hochauflösende Sonographie mit 100 MHz ist mit sehr vielen Störfaktoren behaftet, allein schon dadurch, dass sie als Kontaktverfahren bei unterschiedlichem Schallkopf-Auflagedruck unterschiedliche Dickenmessungen verursacht. Die optische Kohärenz-Tomographie (OCT) könnte aufgrund ihrer hohen Auflösung dafür eine Lösung darstellen. Jedoch haben die bisherigen Erfahrungen mit allen drei Verfahren gezeigt, dass eine Unterscheidung zwischen Schwellung, Entzündung und kanzerogen-pathologischem Befund ("Dignität") nicht allein anhand morphologischer Information möglich ist.

Deshalb ist einer Weiterbildung des erfindungsgemäßen Verfahrens die fluorszenzspektroskopische, ortsaufgelöste Funktionaldiagnose in eine morphologische Darstellung des Untersuchungsgebietes eingebunden. Dazu wird die von der Strahlquelle ausgesandte Strahlung zur zusätzlichen Charakterisierung des Gewebes per Kohärenz-Reflektometrie in einen Anregungsstrahl zur Anregung einer Fluoreszenz in dem Gewebe und einen Referenzstrahl aufgeteilt, wobei der Anregungsstrahl zum Teil vom Gewebe zurückre flektiert und der Referenzstrahl mit der vom Gewebe zurückreflektierten Strahlung überlagert wird.

Besonders bevorzugt weist das der von der Strahlquelle ausgehenden Strahlung aufgeprägte Intensitätsprofil einen charakteristischen Verlauf mit mindestens zwei Intensitätsmaxima auf. Dieser "Fingerprint" des Strahls kann dazu genutzt werden, die optischen Weglängen für den Referenzstrahl und des reflektierten Strahls abzugleichen. Dazu wird in vorteilhafter Weise die optische Weglänge für den Referenzstrahl so eingestellt, dass sich die auf die von der Strahlquelle ausgesandten Strahlung aufgeprägte Intensitätsverteilung im Interferenzdetektor nachweisen lässt, wobei in diesem Fall die optische Weglänge für den Referenzstrahl der vom Anregungsstrahl zurückgelegten optischen Weglänge von der Strahlquelle bis zum Fokus und zurück bis zum Interferenzdetektor entspricht.

Will man Mehrphotonen-Anregungsprozesse mit der optischen Kohärenz-Reflektometrie kombinieren, um so zusätzliche Informationen über den Ort der Anregung zu gewinnen, so ergibt sich das Problem der räumlichen Zuordnung der beiden Prozesse bzw. Informationen. Es besteht besonders im Fall von optisch inhomogenen Proben (z.B. Gewebe) darin, dass der inhomogene Brechungsindex des Materials den Ort der Mehrphotonen-Anregung und das Signal optischen Kohärenz-Reflektometrie in unterschiedlicher Weise beeinflusst: In den Weg des Anregungslichtes geht die Brechkraft bis zum Ort der Anregung (Fokus) in die Tiefe des Materials nur einmal ein. Detektiert die rückgestreuten Signale mit der Methode der optischen Kohärenz-Reflektometrie, so geht in deren Strahlengang der Brechungsindex zweimal ein, indem das Licht auf dem Hin- und auf dem Rückweg das Material durchdringen muss. Somit fehlt für die wünschenswerte Kombination von Mehrphotonen-Anregung und optischer Kohärenz-Reflektometrie die räumliche Zuordnung zueinander insbesondere entlang der Ausbreitungsrichtung der Anregungsstrahlung.

Zusammenfassend gibt es für die Kombination von Fluoreszenzspektroskopie und Kohärenzspektroskopie insbesondere die bevorzugte Weiterbildung

- Dass die Strahlung eines zur Anregung von Zwei- oder Mehrphotonenprozessen und/oder zur optischen Kohärenz-Reflektometrie geeigneten Lasers in einen Anregungs- und einen Refrenzstrahl aufgeteilt wird,
- Dass dem Anregungsstrahl quer zu seiner Ausbreitungsrichtung eine solche Struktur aufgeprägt wird, dass sich nur in der Fokalebene der nachfolgenden Optik oder in deren unmittelbarer Umgebung quer zur Ausbreitungsrichtung des Anregungslichtes eine Intensitätsstruktur mit mindestens zwei Maxima getrennt durch ein Minimum herausbildet,
- Dass dieser Fokalbereich mit jener Region übereinstimmt, in der vorrangig die gewünschten Zwei- oder Mehrphotonenprozesse stattfinden,
- Dass die im Target entstehende rückgestreute Strahlung von der Fokussierungsoptik aufgefangen und sich der ursprünglichen Ausbreitungsrichtung der Anregungslichtes entgegengesetzt ausbreitet,

- Dass auf diesem Weg ein geeignetes optisches Umlenkelement eingebracht wird, ohne dass diese rückgestreuten Strahlung erneut jenes Element durchläuft, das dem Anregungsstrahl seine Struktur quer zu seiner Ausbreitungsrichtung aufgeprägt hat,
- Dass die rückgestreute Strahlung in einer zur der Fokalebene optisch konjugierten Fläche mit dem Referenzstrahl derart zur Interferenz gebracht wird, dass die zu den beleuchteten Punkten des Targets konjugiert liegenden Punkte vom Referenzstrahl vollständig erfasst werden,
- Dass das dort entstehende Interferenzmuster mit einem ortsauflösenden lichtempfindlichen Detektor erfasst und ausgewertet wird,
- Dass die optische Weglänge des Referenzstrahles verändert werden kann, und
- dass als Anzeige für die Übereinstimmung des Ortes der Zwei- oder Mehrphotonenanregung mit jenem Ort, von dem die rückgestreuten optischen Signale stammen, das Bild des Interferenzmusters genommen wird, indem sich jene Struktur widerspiegelt, die nur Im Fokalbereich der Anregungsstrahlung hervortritt.

Der Grundgedanke der Erfindung besteht darin, dass dem Anregungsstrahl eine Struktur aufgeprägt wird, die nur im Fokus - also am Ort der Multiphotonen-Anregung - hervortritt, bzw. sich mit der Ausbreitungslänge derart ändert, dass in der Umgebung des Fokus eine detektierbare Veränderung in dieser Struktur oder genauer ihres Abbildes hervortritt. Die Interferenz des rückgestreuten Lichtes in einer zur Anregungsebene optisch konjugierten Ebene wird genau wird ein Abbild dieser Struktur aufweisen. Mit einem ortsauflösenden Nachweiselement für das Interferenzsignal kann nun der Referenzstrahlengang in seiner Länge angepasst werden, bis die zu erwartende Struktur im Interferenzmuster hervortritt. Dies ist der Indikator für die Überlagerung des Ortes der Multiphotonenanregung mit dem der morphologischen Abtastung mittels optischer Kohärenz-Reflektometrie.

Zusammenfassend bieteten sich insbesondere die folgenden Vorteile:

- Die charakteristisch unterschiedlichen Fluoreszenzkurven von tumorösem und normalem Gewebe erlauben eine Gewebsdiagnose mit signifikanter Selektivität und Spezifität,
- Das Gewebe wird mit einer für das Verfahren dienlichen Fokusgröße im suprazellulären Bereich abgescannt, so dass eine lokale Bewertung des Gewebestoffwechsels in Echtzeit in dienlicher Auflösung möglich wird,
- Als eine vorteilhafte Gruppe von Ausprägungen wird die Größe des abtastenden Fokus von der Größe des einem Scanpunkt zuzuordnenden "Scanvolumens" entkoppelt. Dadurch werden ungünstige Restriktionen erfindungsgemäß aufgehoben, die bei den bisher bekannten optischen Diagnoseverfahren dem hier angestrebten Einsatzzweck im Wege standen.
- Durch besondere Referenzierungsverfahren wird ein lokaler Dignitätskennwert ermittelt ("spektraler Fingerabdruck"), der den Stoffwechselstatus, den Grad der Gefäßneubildung oder den Strukturierungsstatus des betrachteten Gewebsareals weitgehend unabhängig von Störgrößen beschreibt und eine automatische präpathologische Bewertung ermöglicht,
- Die Kombination von räumlich aufgelöster, funktionaler Biostatusbewertung und morphologischer Bildgebung stellt einen qualitativen Sprung in der klinischen Diagnostik der oberen Hautschichten dar,
- Im Besonderen wird durch Parameterwahl und das spezielle Auswerteverfahren des Verfahrens eine ausreichende Eindringtiefe bis zur Basalmembran erreicht, so dass eine Unterscheidung zwischen unterschiedlich invasiven Formen kanzerogener Erscheinungen möglich wird.

Die Strahlquelle wird somit sowohl für die Multiphotonen-Anregung als auch für optische Kohärenz-Reflektometrie eingesetzt. Gegebenenfalls sind die spektralen Eigenschaften der Quelle so zu gestalten, dass sie die gewünschte Tiefenauflösung (Auflösung entlang der Strahlungsausbreitung) ermöglicht. Diese Strahlung wird in einen Anregungsstrahl und einen Referenzstrahl mit einem teildurchlässigen Spiegel geteilt. Beide Strahlen können noch mit Modulatoren versehen werden, die die Intensität nicht jedoch die optische Weglänge zeitlich modulieren.

Eine besonders bevorzugte Variante des Verfahrens sieht vor, dass

a) das Bestrahlen des Gewebes mit Strahlung im Wellenlängenbereich von 720-800 nm erfolgt, wodurch im Gewebe eine Zweiphotonen-Fluoreszenz angeregt wird;
b) Detektieren der Intensität eines ersten Fluoreszenzsignals bei einer Wellenlänge von $460\pm30$ nm und eines zweiten Fluoreszenzsignals bei einer Wellenlänge von $550\pm30$ nm;
c) Bestimmten des Verhältnisses der Intensitäten des ersten und zweiten Fluoreszenzsignals; und
d) in Abhängigkeit von dem in Schritt c) bestimmten Verhältnis ein Signal erzeugt wird.

Dabei ist der Wellenlängebereich bei $460\pm30$ nm signifikant für NAD(P)H und der Wellenlängebereich bei $550\pm30$ nm signifikant für Flavine. Die Bildung des Verhältnisses der Messwerte aus (a) und (b) dient zur Referenzierung der Störfaktoren und Gewinnung eines Aktivitätssignals für die Stoffwechselaktivität, das sich signifikant zwischen normalem und pathologischen Gewebsvolumina gleicher Art unterscheidet.

Eine weitere, bevorzugte Variante sieht vor, dass

a) das Gewebe mit Strahlung im Wellenlängenbereich zwischen 500-550 nm bestrahlt wird, und dabei
b) eine erste Intensität einer Zweiphotonen-Fluoreszenz bei einer Wellenlänge von 340 ±40 nm (signifikant für Tryptophan) detektiert wird;
c) das Gewebe mit Strahlung im Wellenlängenbereich zwischen 720-800 nm bestrahlt wird, und dabei
d) eine zweite Intensität einer Zweiphotonen-Fluoreszenz bei einer Wellenlänge von 460 ±40 nm (signifikant für NAD(P)H) detektiert wird;
e) das Verhältnis der in Schritt b) bestimmten ersten Intensität und der in Schritt d) bestimmten zweiten Intensität bestimmt wird (zur Referenzierung der Störfaktoren und Gewinnung eines Kennwertes, der sich signifikant zwischen normalem und pathologischen Gewebsvolumina gleicher Art unterscheidet); und
f) Erzeugen eines elektrischen Signals in Abhängigkeit von dem in Schritt c) bestimmten Verhältnis.
g) in Abhängigkeit von dem in Schritt f) bestimmten Verhältnis ein elektrisches Signal erzeugt wird.

Eine weitere, bevorzugte Variante des Verfahrens sieht die Schritte vor:

•   Anregung der Zweiphotonen-Fluoreszenz im Wellenlängenbereich 720-800 nm und Messung des Fluorszenzsignals bei 460 ±40 nm [signifikant für NAD(P)H] als Funktion der Gewebetiefe z erfolgt,
•   Speicherung der Messwerte in einem als gesund bekannten Gewebsareal als Funktion der Tiefe (= "Normalverlauf" als Funktion von z) und gleichartige Messung in einem Verdachtsareal (= "Verdachtsverlauf" als Funktion von z),
•   wobei anschließend die beiden Signalverläufe ins Verhältnis gesetzt werden (= "Indikatorverlauf" als Funktion von z), und eine Schranke von 20-40% Abweichung des Indikators vom Wert Eins im Verdachtsareal als Indikationsgrenze für einen anomalen Stoffwechsel verwendet werden, der sich signifikant zwischen normalem und pathologischen Gewebsvolumina gleicher Art unterscheidet.

Des Weiteren kann das Verfahren umfassen:

•   Einstrahlung und Tiefenscan eines fokussierten Kurzpulslaserstrahls im Wellenlängenbereich 720-880 nm oder 980-1080 nm und Messung der remittierten Strahlung bei exakt der halben Einstrahlungswellenlänge [Indikator für Art und Struktur des Kollagens als Strukturmerkmal] als Funktion der Gewebetiefe z in einem in einem als gesund bekannten Gewebsareal als Funktion der Tiefe (= "Normalverlauf" als Funktion von z) und
•   gleichartige Messung in einem Verdachtsareal (= "Verdachtsverlauf" als Funktion von z), wobei
•   anschließend die beiden Signalverläufe ins Verhältnis gesetzt werden (= "Indikatorverlauf" als Funktion von z), und eine Schranke von 30-50% Abweichung des Indikators vom Wert Eins im Verdachtsareal als Indikationsgrenze für eine anomale Gewebestrukturierung verwendet werden, der sich signifikant zwischen normalem und pathologischen Gewebsvolumina gleicher Art unterscheidet.

Dabei kann der fokussierte Strahl wird mittels einer geeigneten (Abtast-) Einrichtung durch das Gewebe bewegt. Diese Bewegung kann in einer der folgenden Abtastmodi ausgeführt werden

a) zweidimensional im Sinne einer horizontalen (= zur Gewebeoberfläche parallelen) Abtastebene,
b) zweidimensional im Sinne einer vertikalen (= zur Gewebeoberfläche senkrechten) Abtastebene ("optischer Ultraschall")
c) dreidimensional im Sinne eines Abtastvolumens.

Ziel ist insbesondere das Auffinden von Tumoren im Frühstadium ("Carcinoma in situ") und die Abgrenzung gegen weiterentwickelte Stadien (Einwandern von Micro-Gefäßen, Durchbrechen der Basalmembran, Verlust der Gewebestrukturierung). Dabei werden folgende Erkenntnisse genutzt

a) Micro-Zirkulation wird am erhöhten Hemoglobin-Signal erkannt.
b) Auflösung der Struktur wird am lokal abweichenden Gehalt an Elastin und/oder Kollagen erkannt.
c) Das Durchbrechen der Basalmembran wird durch Fehlen der Papillenstruktur durch Bildauswertung erkannt.

Zur Unterdrückung von Störungen und zur Kompensation von Einflüssen unbekannter Skalierung einerseits der Messanordnung und andererseits der biologischen Materialien im Anregungs- und Mess-Strahlengang wird mit einer Referenzierung des Mess-Signals gearbeitet, bei der die Messdaten mindestens zweier signifikanter Stoffe herangezogen werden, hier insbesondere der Stoffgruppen NAD(P)H, Flavine und Tryptophan.

Als weitere signifikanzsteigernde Information werden morphologische Daten, wie der lokale Gehalt an Elastin oder Kollagen herangezogen, die sich durch eine eigene Fluoreszenzmerkmale Elastin) oder durch nichtlineare Erzeugung eines Referenzsignals (bevorzugt SHG = Second Harmonic Generation = Frequenzverdoppelung der Anregungsstrahlung, bevorzugt bei Kollagen) nachweisen lassen.

Dem Arzt wird ein morphologisches Bild des untersuchten Gewebeareals am Monitor gezeigt. Dieses beruht auf der Auswertung der Signale der Strukturmoleküle (Elastin und/oder Kollagen) und der direkten Rückstrahlung der Anregungsstrahlung (Oberflächen-Reflex und Streuamplitude).

Ein "Gewebe-spektroskopischer Fingerabdruck" wird durch die genannten Referenzierungen ermittelt und vom Rechner automatisch bewertet [zumindest zweiwertig (= "normal" oder "abweichend") oder dreiwertig (= "normal", abweichend" oder "unklar/unbestimmt")]. Die Bereiche abwechselnden Stoffwechsels werden im Monitorbild in Falschfarbendarstellung wiedergegeben.

Geräteaufbau:

Das Gerät besteht aus einem Betriebsgerät mit Netzversorgung, Bedienelementen integriertem Rechner und angeschlossenem Monitor sowie dem Handstück mit Fokussier-Optik und Scanner für die Anregungsstrahlung und den Elementen zum Sammeln der Mess-Strahlung (reflektierte und Streustrahl bei der Anregungswellenlänge, SHG-Strahlung bei der halben Anregungswellenlänge sowie der Fluoreszenzstrahlung).

Die Zuführung der Anregungsstrahlung geschieht entweder mittels Faseroptik, bevorzugt als Photonic Fibers, oder einem Gelenkarm. Dabei können Elemente der Zuführungsoptik für die Fluoreszenz-Anregungsstrahlung auch zum Sammeln der Mess-Strahlung verwendet werden.

Als eine besonders vorteilhafte Ausführungsform wird bei dem analog zum bildgebenden medizinischen Ultraschall ausgeprägten Abtastmethodik ("Optischer Ultraschall") eine im Bezug zum Handstück fixe Abtastebene vertikal in das Gewebe gelegt und vom Arzt, wie beim Ultraschall üblich durch das Gewebe bewegt. Das Schnittbild des Gewebes mit den genannten Zusatzinformationen ("Fingerabdruck" mit automatischer Bewertung) wird in Echtzeit im Bildschirm angezeigt.

Bei Betriebszuständen mit hoher Auflösung, wo die händische Positionierungsgenauigkeit nicht ausreicht oder die Unruhe durch die unwillkürliche Handbewegung für das Verfahren zu groß ist, wird das Handstück fest aufgesetzt und die Verschiebung der Abtastebene durch elektromotorische oder mikromechanische Stellelemente ausgeführt.

Als Strahlquelle wird bevorzugt ein Laser, insbesondere ein gepulster Laser eingesetzt. Eine mögliche Anregungsquelle, die mit Pulsen im Pikosekundenbereich arbeitet, erlaubt zudem durch die Verwendung einer photonischen Faser auch die Erzeugung breitbandigen Lichtes zur Anregung bei mehreren Farben. Eine derartige Lichtquelle ist bislang weder in der fluoreszenzbasierten Tumordiagnostik noch in der Mehrphotonenmikroskopie eingesetzt worden.

Das Verfahren grenzt sich von den bekannten Diagnoseverfahren dadurch ab, dass es die Dignität des Gewebezustandes in einem dreidimensionalen Scanbild der Haut bewertet und darstellt. Dabei ist die Auflösung so gewählt, dass -je nach betrachteter Gewebstiefe- eine Anzahl von ca. 20 bis 200000 Zellen zum lokalen Signal beitragen.

Damit grenzt sich das Verfahren von höher auflösenden Verfahren ab, die Gestalt, Teilungsverhalten und Stoffwechsel einzelner Tumorzellen mikroskopisch betrachten. Diese liefern labortechnisch und für die Forschung interessante Ergebnisse, sind jedoch für eine klinische Anwendung am Patienten nur sehr bedingt geeignet. Das für die genann ten Laborverfahren notwendige hohe Auflösungsvermögen im Bereich von 1 $\mu$m erfordert ein Epi-Fluoreszenzmikroskop mit hoch-aperturiger optische Abbildung, was ohne Immobilisation des Patienten nicht möglich ist. Ferner ist die erreichbare Gewebstiefe deutlich limitiert. Um die hoch-detaillierte Information zu gewinnen und auszuwerten, benötigt man vergleichsweise lange Messzeiten, weshalb ein solches Verfahren für ein klinisches Staging -anders als das erfindungsgemäße Verfahren ungeeignet ist.

Das oben beschrieben erfindungsgemäße Verfahren arbeitet vollkommen nicht-invasiv. Die Hautoberfläche wird nicht angetastet, so dass auch bei positivem Befund keine Gefahr besteht, durch das Verfahren das Ausschwemmen von Tochterzellen des Tumors auszulösen.

Die Kombination des Fluoreszenzverfahrens mit einem OCT-System liefert wegen des dreidimensionalen Scanvorgangs ein 3D-OCT, so dass simultan dasselbe Gewebsvolumen ("Scan-Volumen") einerseits in dienlicher Auflösung auf seine Dignität bewertet und andererseits morphologisch abgebildet wird. Der untersuchende Arzt erhält daher eine vollständige dreidimensionales Bild des Gewebes und seines bewerteten Stoffwechselzustandes.

Das Verfahren wurde in Bezug auf Haut als relativ leicht zugänglichen Organ dargestellt. Es weist jedoch deutliches Potential zur Weiterentwicklung für andere medizinische und labortechnische Anwendungsgebiete auf.

Wesentlich bei der Erzeugung von Teilstrahlen ist, dass in jedem Teilstrahl die Bedingungen derart eingestellt werden, dass mit der gewählten Optik im Fokus die gewünschte Intensität der Anregungsstrahlung erreicht wird und dass die Teilstrahlen derart eingestellt werden, dass jeder von ihnen mit der gewählten Fokussierungsoptik einen von den Foki der anderen Teilstrahlen getrennten Fokus in der Bildebene der Optik ausbildet. Des Weiteren wird der Abstand dieser mindestens zwei Foki in der Bildebene derart eingestellt wird, dass deren Überlagerung entlang der Ausbreitungsrichtung der Anregungsstrahlung insbesondere in der Nähe des Fokus jedes Teilstrahles einen wählbaren Bruchteil der Intensität der Anregungsstrahlung im Fokus nicht überschreitet.

Zusammenfassend ist bevorzugt vorgesehen, dass

- das optische Element zur Aufteilung in die gewünschte Anzahl von Teilstrahlen derart als Stellelement ausgeführt wird, dass die Anzahl der Teilstrahlen und der Abstand ihre Foki gewählt werden kann dass mit einem geeigneten optischen Element die Teilstrahlen oder der Gesamtstrahl in ihrer Intensität derart modifiziert werden können, dass für die Intensitäten im Fokus jedes Teilstrahles entsprechend den Erfordernissen des Zwei- oder Mehrphotonenanregungsprozesses ein geeigneter Wert eingestellt werden kann,
- dass das optische Element zur Aufteilung in die gewünschte Anzahl von Teilstrahlen oder ein zusätzliches optisches Element eine mit der Zeit veränderbare Ablenkung oder Anordnung der Teilstrahlen ermöglicht, die in der Bildebene des Objektives eine entsprechende mit der Zeit veränderte Variation des Anregungsgebietes oder Teilen davon ermöglicht,
- dass durch eine solche Anordnung die entstehende zeitliche Veränderung des Anregungsgebietes dazu genutzt wird, die durch die Anordnung der Teilstrahlen entstehenden Lücken des Anregungsgebietes in der Fokalregion auszugleichen.
- dass die Formgebung des Anregungsvolumens durch "Microwobbling" erzielt wird
- dass die gewünschten Bewegungen durch mechanische oder elektromagnetische Effekte, wie Pockels- oder Kerreffekt erzielt wird dass mikromechanische Elemente zur schnellen Mikrobewegung des Fokus (oder zur Phasensteuerung verwendet werden.

Die Erfindung betrifft auch eine Vorrichtung zur Durchführung eines wie oben beschriebenen Verfahrens, mit:

- einer Strahlquelle zum Aussenden gerichteter Strahlung zum Bestrahlen des zu charakterisierenden Gewebes, die konfiguriert ist, im Gewebe eine für das Gewebe charakteristische Rückstreustrahlung anzuregen, wobei
- in das Gewebe eingedrungene Strahlung innerhalb eines sich quer zu ihrer Ausbreitungsrichtung erstreckenden Anregungsbereiches eine ausreichende Intensität aufweist, um im Gewebe eine charakteristische Rückstreustrahlung anzuregen, und
- Strahlformungsmitteln, die konfiguriert sind, der von der Strahlquelle ausgesandten Strahlung ein derartiges Intensitätsprofil aufzuprägen, dass der Anregungsbereich eine Mehrzahl von Zellen des Gewebes überdeckt und die beim Bestrahlen des Gewebes angeregte Rückstreustrahlung auf zellübergreifende Gewebeeigenschaften zurückgeht, wobei
- die Strahlformungsmittel derart ausgebildet sind, die von der Strahlquelle ausgesandte Strahlung in mindestens zwei Teilstrahlen aufzuteilen, wobei der Anregungsbereich durch die Lage der Teilstrahlen festgelegt ist, wobei
- Abbildungsmittel vorhanden sind, die derart ausgebildet sind, die Teilstrahlen jeweils so in das Gewebe zu fokussieren, dass die Fokuspunkte für die Teilstrahlen beabstandet zueinander sind; und
- einem Detektor zum Detektieren der Rückstrahlung, der in Abhängigkeit von der detektierten Strahlung ein elektrisches Signal erzeugt und der derart konfiguriert ist, die Rückstrahlung summarisch aus den Fokalbereichen aller in das Gewebe fokussierten Teilstrahlen zu erfassen.

Für einen sicheren Betrieb einer Vorrichtung zur optischen Gewebeuntersuchung gibt es folgende grundsätzliche Forderungen:

A. Im regulären Betrieb dürfen keinerlei Schädigungen für den Patienten auftreten

B. Im "Ein-Fehler-Fall" dürfen ebenfalls keine Schädigungen für den Patienten eintreten

C. Für den ungünstigsten anzunehmenden Fall aus der Kombination mehrerer Fehlfunktionen werden die Folgewirkungen diskutiert. Eine Schädigung des Patienten mit Krankheitswert muss ausgeschlossen sein.

Die drei Forderungen werden in den folgenden Abschnitten als Risikoanalyse gemäß ISO 14191 separat bewertet. Ein anschließendes Fazit fasst die Sicherheitsbewertung zusammen.

Beschreibung des regulären Betriebes (Fall A):

Kategorien:

Im Regelbetrieb führt eine erfindungsgemäße Vorrichtung eine Scanbewegung aus, indem der Fokus des Lasers lateral und in der Tiefe durch das Gewebe bewegt und durch eine Intensitätsregelung die Abschwächung des Strahles im Gewebe ausgeglichen und die Intensität im Fokus konstant gehalten wird. Aus Sicherheitsgründen muss gefordert werden, dass einerseits ein Einzelpuls keine Schädigung verursacht. Andererseits darf die überlagerte Wirkung der Folgepulse sowie die während der Anwendung im Gewebe akkumulierte Energie nicht zu Schädigungen führen.

Beim Einzelpuls müssen adiabatische thermische Effekte, der Einfluss der Photochemie sowie intra- und intermolekulare Übergangszeiten aufgrund der schnellen energetischenAnregungsvorgänge betrachtet werden. Da die höchste Anregungsintensität im Fokus der Laserstrahlung liegt, sind die kennzeichnenden Größen hier die Volumendichte der absorbierten Pulsenergie und der absorbierten Pulsspitzenleistung im Fokusvolumen. Wärmeleitungsvorgänge können außer Betracht bleiben.

Für die Bewertung der überlagerten Wirkung der Folgepulse ist die absorbierte Energie der Einzelpulse auf das durch die Scanbewegung überstrichene Volumen zu beziehen. Für den Fall, dass sich die Anregungsvolumina der Einzelpulse überlappen, kann es hierbei zu einer höheren Belastung als durch einen einzelnen Puls kommen.

Während in den beiden obigen Fällen die Absorption der Strahlung im Fokusvolumen betrachtet wird, muss zur Beurteilung der Wirkung der im Gewebe akkumulierten Energie die starke Streuung des Gewebes berücksichtigt werden. Sie führt dazu, dass selbst im Wellenlängenbereich des optischen Fensters bei vergleichsweise geringer Absorption die Strahlung ein begrenztes Gewebevolumen nicht verlässt und letztlich in diesem Volumen absorbiert wird. Kennzeichnend ist hier die mittlere Leistung sowie Wärmetransportvorgänge im Gewebe, d.h. die Wärmeleitung und Wärmeabtransport durch Blutzirkulation sind zu berücksichtigen.

Nichtthermische Wirkungen:

Aufgrund der geringen Photonenenergie ($\sim 10^4$ cm$^{-1}$) und Leistungsdichte ($\sim 10^{11}$ W/cm$^2$) können Schädigungen durch Bindungsbruch ausgeschlossen werden. Schädigungen durch Temperaturgradienten bei Erwärmung im ps-Zeitbereich sind nicht bekannt, daher konzentriert sich die Abschätzung der Gewebeschädigung im Folgenden auf die rein thermische Belastung.

Abschätzung und Bewertung der thermischen Belastung des Gewebes:

Bei der Bewertung der schädlichen Nebenwirkungen müssen sowohl die Absorptions- als auch die Streueigenschaften des Gewebes berücksichtigt werden. In dem vorgesehenen Wellenlängenbereich der Strahlung ist der Wirkungsquerschnitt für Streuprozesse um ein bis zwei Größenordnungen höher, als der für die Absorption des Lichtes. Für 1064 nm wird ein Absorptionskoeffizient von 4 cm$^{-1}$ für die reine Absorption und 5 cm$^{-1}$ als effektiver Wirkungsquerschnitt für die Kombination aus Absorption und Streuung angegeben. Die Eindringtiefe (Abfall der Intensität auf 1/e) wird mit 4 mm beziffert. Temperaturänderungen ergeben sich aufgrund der kurzen Pulsdauer ($\sim 1$ ps) zunächst adiabatisch aus der absorbierten Energie geteilt durch die Wärmekapazität des Gewebes. Als konservative Abschätzung soll hier der kleinste Wert für die Wärmekapazität (1930 J kg$^{-1}$ K$^{-1}$ für Fettgewebe) angenommen werden. Die Temperaturunterschiede gleichen sich dann für längere Zeiten durch die Wärmeleitfähigkeit des Gewebes wieder aus. Auch hier wird für die Abschätzung der kleinste Wert ($\lambda = 0.3$ W m$^{-1}$K$^{-1}$ in Fett) angenommen.

Für die Abschätzung der adiabatischen Wirkung eines Einzelpulses reicht es, den Ort der maximal auftretenden Intensität zu betrachten. Dieser liegt im Fokus, wo sich die gesamte dort während eines Laserpulses absorbierte Energie adiabatisch, d.h. ohne Wärmeaustausch mit der Umgebung, akkumuliert. Aufgrund der Intensitätsregelung ist diese Intensität für alle Scanpositionen, d.h. für alle Tiefen im Gewebe, identisch. Ein Energieübertrag geschieht nur durch lineare und nichtlineare Absorption; Streuung braucht in der Rechnung nicht berücksichtigt zu werden. Unter der Annahme einer Absorption von 50% pro mm und der o.g. Wärmekapazität ergibt sich aus den vorgesehenen Geräteparametern eine Erwärmung des Fokusvolumens um 2.6 °C durch einen einzelnen Laserpuls.

Für die Bewertung der überlagerten Wirkung der Folgepulse während des lateralen Scanvorganges ist z.B. die absorbierte Energie aller Pulse zu berücksichtigen, die während 100 μm Scanstrecke auf das Gewebe auftreffen. Diese ist auf das entsprechende Scanvolumen (Breite: Taillendurchmesser, Tiefe: doppelte Rayleighlänge 100 μm) zu beziehen. Als konservative Abschätzung soll auch diese Betrachtung adiabatisch durchgeführt werden, obwohl es in dem Zeitraum zwischen zwei Pulsen zu einer geringfügigen Verteilung der eingetragenen Wärme in Gewebe kommt. Die Berechnung mit o.g. Werten ergibt eine Erwärmung des Gewebes um 4.7 °C im Fokalbereich des Lasers bei einmaligem lateralen Überstreichen mit dem Messfleck. Da der Scanvorgang 'zeilenweise' erfolgt, d.h. der Messfleck erst in einer festen Tiefe lateral durch das Gewebe bewegt wird, bevor in einer neuen Tiefe die nächste 'Zeile' gemessen wird, ist die überlagerte Wirkung der Pulse in der Tiefe nicht adiabatisch abzuschätzen. In dem Zeitraum zwischen zwei 'Zeilen' findet eine Diffusion der Wärme in ein größeres Volumen statt, sodass die überlagerte Wirkung zweier saggital benachbarter Pulse durch die im folgenden Abschnitt betrachtete gemeinsame Wirkung aller Pulse bei weitem übertroffen wird.

Für die Bewertung dieser langfristig (d.h. über mehr als $10^5$ Pulse) im Gewebe akkumulierten Energie ist diese auf das Volumen zu beziehen, das die Strahlung aufgrund der starken Streuung nicht verlässt. Als konservative Abschätzung wird die höchste Laserleistung, d.h. für die Messung in größter Tiefe, angenommen. Geht man von einem zylindrischen Streuvolumen mit 2 mm Durchmesser und 4 mm Tiefe aus, dessen Zentrum lateral schnell auf einer Linie von 4 mm bewegt wird, ergibt sich eine Erwärmung um 1 °C pro Sekunde in diesem Volumen. Unter stationären Bedingungen, d.h. wenn der Applikator auf der Haut lange nicht bewegt wird, begrenzt der Wärmetransport des Gewebes unter ungünstigsten Bedingungen (Fettgewebe, s.o.) die Temperatur auf 43 °C in der Strahlebene (10 $\mu$m Dicke). (Annahme: Diffusive Wärmeleitung, in 5 mm Entfernung wird die Körpertemperatur durch Blutzirkulation auf 37 °C gehalten).

Um die sicherheitstechnische Bedeutung einer Temperaturerhöhung im Gewebe zu bewerten, kann auf Literaturergebnisse zurückgegriffen werden (siehe zum Beispiel G. Müller, H. P. Berlien "Angewandte Lasermedizin", ECOMED-Verlag, Loseblattsammlung, 1993 ff., Kapitel 3.3 "Thermische Wirkungen"). Demnach ist eine schädigende thermische Wirkung sowohl von der Temperatur, als auch von deren Einwirkdauer abhängig. Bei den hier betrachteten Zeitskalen sind die Grenztemperaturen für unter 1 bis maximal 10 Sekunden anzusetzen. Schäden treten bei diesen Einwirkdauern gemäß den Literaturdaten erst bei über 57°C ein, also 20K über der Körpertemperatur!

Beschreibung des Ein-Fehler-Falles (Fall B):

Folgende Fehlerquellen sind aus Sicherheitsgründen zu betrachten:

  a) Die Pulsenergie erhöht sich auf den Maximalwert des Lasers durch Fehler der Regelung
  b) Der Scanvorgang unterbleibt, sodass die Folgepulse dieselbe Stelle wiederholt treffen.

Fehlerbehandlung: Als wichtigste Maßnahme gegen die beiden unzulässigen Betriebszustände a) und b) ist in der Konstruktion und Systemlayout eine schnelle Überwachungselektronik vorgesehen, die den Regelbetrieb sowie den augensicheren Kontakt des Applikators auf der Haut ständig überprüft und die ihrerseits unabhängig von der Steuerelektronik des Gerätes arbeitet. Sobald die Überwachungselektronik einen Fehler erkennt, schaltet sie die Laserquelle ab. Dadurch ist die Gefahr beseitigt. Als zusätzliche Maßnahme wird für den Fall a) eine Hardwarelimitierung in den Laser eingesetzt, die eine Energieabgabe über bestimmte Werte hinaus verhindert.

Bewertung des Restrisikos (Fall C):

Eintrittswahrscheinlichkeit der Fehler:Die Eintrittswahrscheinlichkeit der genannten Fehler (Pulsenergie erhöht sich, Scanvorgang unterbleibt) wird durch elektronische Maßnahmen und die unabhängige Überwachungselektronik auf einen akzeptablen Wert abgesenkt (genaue Definition kann erst bei Erstellung der Anlage angegeben werden).

Folgewirkung des Mehr-Fehler-Falles:

Sollte trotzdem ein Fehler in der Überwachung auftreten, so sind die Auswirkungen wie folgt zu beurteilen:

  a) Ausfall der Leistungsregelung: Die Wirkung der Einzelpulse sowie die überlagerte Wirkung der Folgepulse wurde für den Regelbetrieb adiabatisch berechnet. Daher skaliert die Wirkung (unter Vernachlässigung der nichtlinearen Absorption) linear mit der Laserleistung. Damit bleibt die kurzzeitige adiabatische Erwärmung unterhalb 10 °C. Für die Bewertung der im Gewebe akkumulierten Energie wurde bereits für die Abschätzung im Regelfall mit der maximalen Laserleistung gerechnet. Folglich führt ein Ausfall der Leistungsregelung nicht zu einer Schädigung des Patienten.

  b) Ausfall der Scanbewegung: Die Einzelpulse werden alle in dem selben Gewebevolumen fokussiert, was zu einer schnellen Temperaturerhöhung führt, bis das Fokusvolumen durch die Wärmediffusion ins umliegende Gewebe eine konstante Temperatur von 62 °C erreicht. Für die im Gewebe akkumulierte Leistung bedeutet ein Ausfall der Scanbewegung, dass sich eine maximale Temperatur von 50 °C auf der Achse des Laserstrahles bis in eine Tiefe von ca. 4 mm einstellt. Folglich führt ein Ausfall der Scanbewegung ohne Abschaltung des Lasers zu einem Schmerzreiz bei dem Patienten. Dies deckt sich mit Selbstversuchen, in denen diese Strahlungsbedingungen an empfindlichen Hautregionen (Ellenbeuge) als "Nadelstiche" wahrgenommen wurden.

Es ist anzumerken, dass ein Ausfall der Scanbewegung die Bilderzeugung verhindert und der behandelnde Arzt daher die Störung erkennt. Bereits ein Abheben des Applikators von der Haut wird zur sofortigen Abschaltung des Lasers führen.

Die Erfindung wird im Folgenden anhand von Ausführungsbeispielen unter Bezugnahme auf die Figuren näher erläutert. Es zeigen:

Fig. 1      schematisch eine Vorrichtung zur optischen Gewebecharakterisierung gemäß einer ersten Ausführungsform der Erfindung;

Fig. 2      beispielhaft den Verlauf der Intensität für verschiedene Intensitätsprofile;

Fig. 3      schematisch eine Vorrichtung zur optischen Gewebecharakterisierung gemäß einer zweiten Ausführungsform;

Fig. 4A, 4B      schematisch eine dritte und vierte Ausführungsformen einer Vorrichtung zur optischen Gewebecharakterisierung;

Fig. 5      eine fünfte Ausführungsformen einer Vorrichtung zur optischen Gewebecharakterisierung;

Fig. 6      Graphische Darstellung eines Ergebnis einer Fluoreszenzmessung;

Fig. 7      Graphische Darstellung der Intensität einer in einem Gewebe angeregten Ein-Photonen-Fluoreszenz;

Fig. 8      Graphische Darstellung der Intensität einer in einem Gewebe angeregten Zwei-Photonen-Fluoreszenz;

Fig. 9      eine sechste Ausführungsformen einer Vorrichtung zur optischen Gewebecharakterisierung;

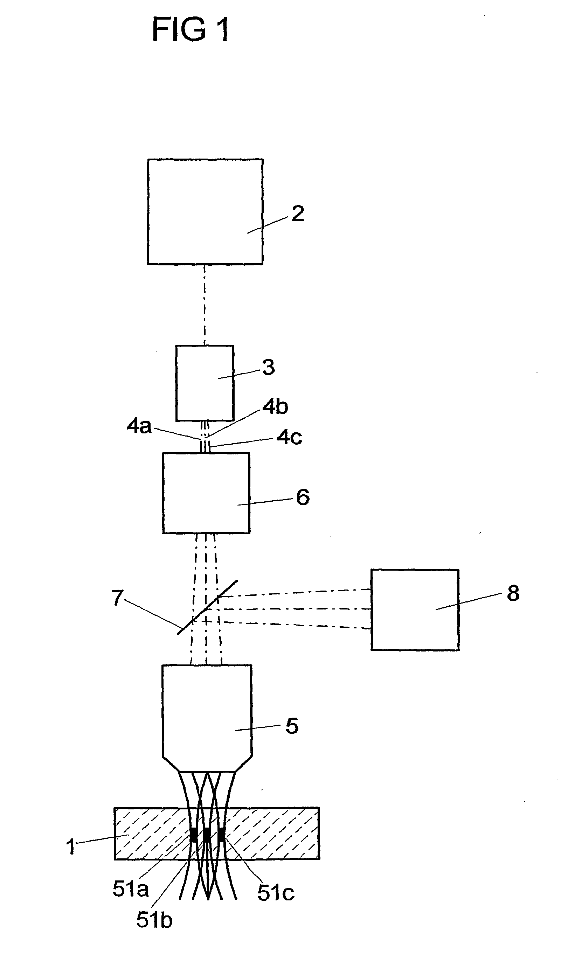

Die Fig. 1 zeigt eine Vorrichtung zur optischen Charakterisierung eines menschlichen oder tierischen Gewebes 1. Das Licht eines für die Auslösung von Mehrphotonenprozessen typischerweise eingesetzten Lasers 2 wird mit einem Strahlteiler 3 in Teilstrahlen 4a, 4b, 4c aufgeteilt. Es sind drei Teilstrahlen dargestellt. Es versteht sich jedoch, dass die Erfindung nicht auf drei Teilstrahlen beschränkt ist, sondern eine beliebige Anzahl von Teilstrahlen verwendet werden kann.

Deren unterschiedliche Richtungen der Teilstrahlen 4a, 4b, 4c werden durch ein Objektiv 5 in eine entsprechende Anordnung von Foki 51a, 51b, 51c umgesetzt. Auf dem optischen Weg zwischen der Strahlaufteilung und dem Objektiv 5 können einerseits Elemente zur Beeinflussung z.B. der Intensität der Teilstrahlen eingebracht werden (Hier nicht dargestellt) oder - wie in der Fig. 1 beispielhaft dargestellt - ein Scanner 6, der zur abrasternden Anregung des Targets 1 (Gewebe) benutzt wird.

In den Foki 51a, 51b, 51c der Teilstrahlen 4a, 4b, 4c findet auf Grund der hohen Intensitäten eine Mehrphotonen-Absorption statt, in deren Folge diese Bereiche ein entsprechendes Fluoreszenzsignal aussenden, das zur Charakterisierung des bzw. der Anregungsbereiche(s) herangezogen werden kann. Dieses Fluoreszenzlicht wird zu einem Teil von dem Objektiv 5 erfasst, durch einen dichroidischen Spiegel 7 von dem Anregungslicht spektral und räumlich getrennt und mit einem Fluoreszenzdetektor 8 nachgewiesen.

Die Fig. 1 macht deutlich, dass durch die summarische Erfassung dieses Fluoreszenzsignales der gesamte Bereich ("integraler Anregungsbereich") charakterisiert wird, der von dem Bündel der Anregungsfoki im Target überdeckt wird. Sollte es aus nachweistechnischen Gründen erforderlich sein, auch die Lücken in diesem Anregungsmuster zu erfassen, so kann dies beispielsweise durch minimale Scanbewegungen und die Mittelung über mehrere Laserpulse erfolgen.

Eine solche Anordnung ist insbesondere dann von Vorteil, wenn zum Beispiel um Schäden zu vermeiden, die Intensität der Anregungsstrahlung nach oben hin begrenzt ist. In der vorgeschlagenen Anordnung kann jeder Teilstrahl mit der maximal zugelassenen Intensität beaufschlagt werden. Entsprechend der Zahl der gewählten Teilstrahlen wächst dann das zu erwartenden Fluoreszenzsignal gegenüber dem von einem Teilstrahl. Dabei kann man jedoch - wie einführend beschrieben - die räumliche Begrenzung des Anregungsgebietes entlang der Ausbreitungsrichtung des Anregungsstrahles und damit die Verfahrensauflösung weitgehend unverändert beibehalten.

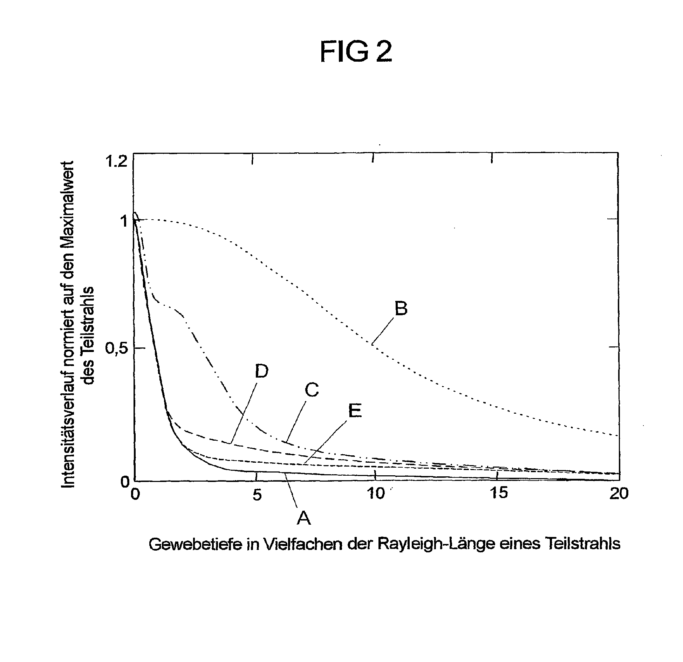

Die erfindungsgemäß wichtige Fragestellung der geeigneten Anordnung der Foki im Target wird anhand der Fig. 2 erläutert. Die Fig. 2 zeigt für den ausgewählten Fall der Überlagerung von neun Teilstrahlen gleicher Intensität die Abhängigkeit des normierten Intensitätsverläufe im Zentrum des Bündels über der Achse der Ausbreitungsrichtung der Strahlung in Vielfachen der Rayleigh-Länge eines Teilstrahles. Für die Fokalebene ist z zu Null gesetzt.

Dabei zeigt die durchgezogene Linie A den Verlauf für einen Teilstrahl. Zum Vergleich zeigt die gepunktete Linie B den Intensitätsverlauf für einen Strahl mit gleicher Spitzenintensität wie in A, jedoch - durch entsprechend gewählte Apertur - mit der gleichen Gesamtenergie wie die neun Teilstrahlen. Diese Kurve weist ein deutlich langsameres Abklingen der Intensität längs der Ausbreitungsrichtung auf. Dies bedeutet, dass das Anregungsgebiet in dieser Achse deutlich verlängert wäre.

Die anderen drei Kurven stellen den Intensitätsverlauf für unterschiedliche laterale Abstände der Foki der Teilstrahlen dar (C: Abstand = 1,5-faches des Taillenradius; D: Abstand = Dreifaches des Taillenradius; E: Abstand = Fünffaches des Taillenradius).

Wählt man den Abstand zu klein (z.B. 1,5-fache des Taillenradius), so ergibt die Überlagerung etwas höhere Intensitäten im Fokus selbst und das Anregungsgebiet ist entlang der Ausbreitungsrichtung deutlich vergrößert. Man erkennt dies, wenn man beispielsweise den Punkt des Abklingens auf die halbe Fokusintensität als Maßstab nimmt.

Stellt man jedoch beispielsweise den drei- oder fünffachen Taillenradius in dieser Beispielanordnung ein, so ist die Intensität annähernd im gleichen Abstand vom Fokus auf die Hälfte abgefallen wie im Fall eines Strahles mit gleicher Taille.

Das Beispiel macht deutlich, dass es durch erfindungsgemäß vorteilhafte Wahl des Abstandes der Foki gelingt, die Ausdehnung des Anregungsgebietes entlang der Ausbreitungsrichtung nahezu unabhängig von der Zahl der Teilstrahlen und damit von der Größe des Anregungsgebietes zu gestalten.

Die Wahl des günstigsten Abstandes der Foki kann von ihrer Anordnung in der Fokusebene des Objektiv, ihrer Zahl und den Eigenschaften der Anregungsstrahlung abhängen. Bevorzugt wird ein Abstand in der Größe des 2,5 -15-fachen des Taillenradius eines Einzelstrahls gewählt.

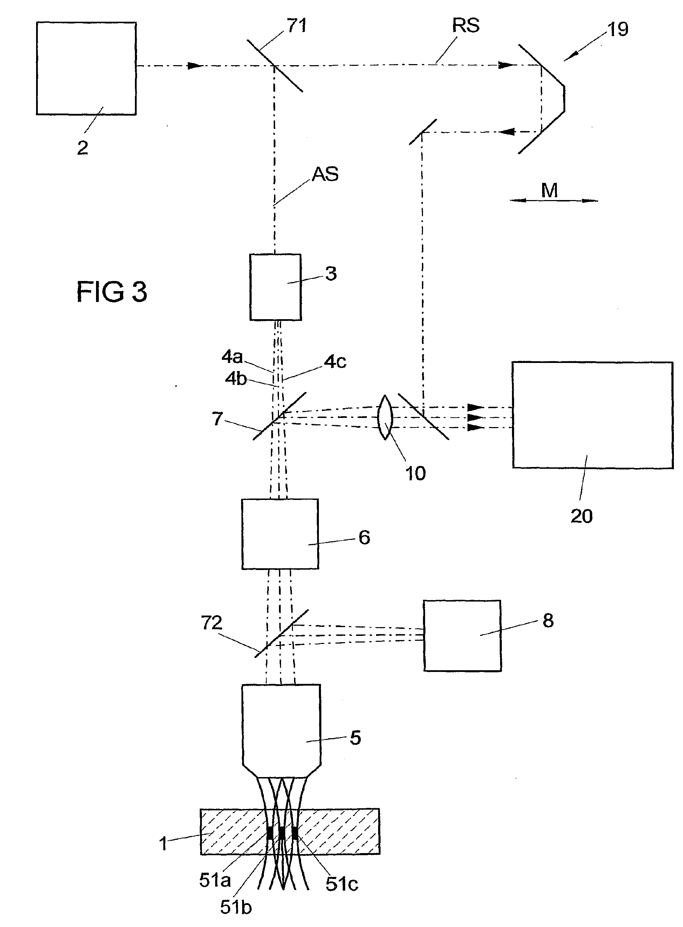

Die Fig. 3 zeigt eine Vorrichtung, in der eine Laser-Strahlquelle 2 sowohl für die Multiphotonen-Anregung als auch für optische Kohärenz-Reflektometrie eingesetzt wird. Gegebenenfalls sind die spektralen Eigenschaften der Quelle so zu gestalten, dass sie die gewünschte Tiefenauflösung (Auflösung entlang der Strahlungsausbreitung) ermöglicht. Diese Strahlung wird in einen Anregungsstrahl AS und einen Referenzstrahl RS mit einem teildurchlässigen Spiegel 71 geteilt.

Beide Strahlen können noch mit Modulatoren versehen werden, die die Intensität nicht jedoch die optische Weglänge zeitlich modulieren. Eine solche Anordnung ist nicht abgebildet.

Dem Anregungsstrahl AS wird quer zur Ausbreitungsrichtung in der Strukturierungseinheit 3 eine Struktur aufgeprägt, die infolge der Fokussierung nur in der Nähe der Fokalebene eines Objektivs 5 innerhalb des Targets kontrastreich hervortritt. Für diese Einheit können beispielsweise ein holographisches Element oder Mikrolinsen- oder Mikroprismen-Anordnungen eingesetzt werden. In der Fig. 3 ist als mögliche Ausführung einer solchen Anordnung die Aufteilung in drei Teilstrahlen 4a, 4b, 4c dargestellt, die in dem Target 1 (Gewebe) eng benachbarte Foki ausbilden. Außerhalb der Fokalebene verringert sich der Kontrast zwischen den Foki infolge der Durchmischung der Teilstrahlen. Der so strukturierte Anregungsstrahl AS kann mit einem Scanner 6 zur ortsdefinierten Anregung des Targets 1 relativ zu diesem bewegt werden.

Dort wo der strukturierte Anregungsstrahl AS das Innere des Targets 1 beleuchtet, findet an Strukturgrenzen Streuung statt. Der rückgestreute Anteil durchläuft das Objektiv 5, in dem er wieder kollimiert wird, und gegebenenfalls den Scanner 6, der die abtastende Strahlbewegung rückgängig macht. Über einen Strahlteiler 7 und gegebenenfalls eine Feldlinse 10 gelangt dieses aus der Tiefe des Targets 1 stammende Licht zur Interferenz mit dem Referenzstrahl RS in einem Interferenzdetektor 20 (z.B. eine CCD-Kamera).

Die optische Weglänge des Referenzstrahles RS kann über eine bewegliche Spiegelvorrichtung 19 (Die Bewegungsrichtung ist durch den Doppelpfeil M angegeben) verändert werden. Beobachtet man die Interferenz ortsaufgelöst, so wird man dann ein besonders kontrastreiches Interferenzmuster beobachten, wenn die optische Weglänge des Referenzstrahles genau dem Strahlweg des Anregungslichtes bis zum Fokus und des rückgestreuten zurück bis zum Ort der Überlagerung entspricht. In den Interferenzmuster wird sich das Abbild der dem Anregungsstrahl aufgeprägten Struktur zeigen. Dies ist der Indikator dafür, dass die Kohärenz-Reflektometrie den Fokalbereich der Anregung darstellt.

Als Beispiel ist in der Fig. 3 die Zwei- oder Mehrphotonen-Mikroskopie als möglicher Anwendungsfall dargestellt. In dem hier gezeigten Beispiel wird die Mehrphotonen-Fluoreszenz mittels des dichroitischen Strahlteilers 72 und des Empfängers 8 summarisch aus den Fokalbereichen aller drei Teilstrahlen erfasst.

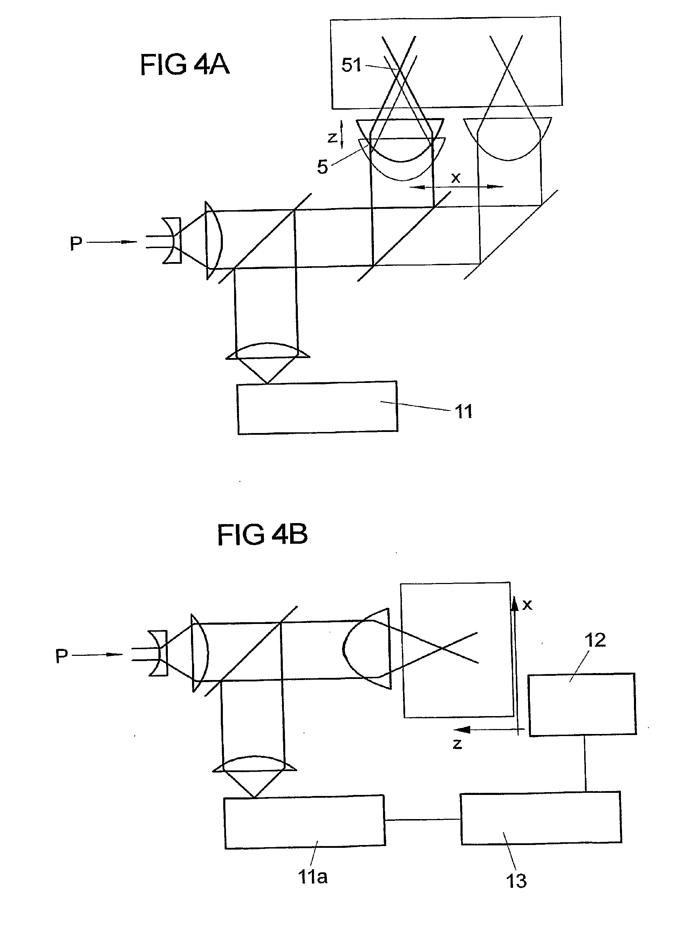

Die Fig. 4A, 4B zeigen das Prinzip eines Seriengerätes zur optische Gewebecharakterisierung. Nach der Anregung durch fokussierte IR-Laserpulse (~ fs bis ps) (Pfeil P) wird das von der Probe / dem Gewebe ausgehende Licht mit einem Spektrometer 11 detektiert. Dieses Spektrometer kann zur Vereinfachung der Auswertung und zur Verbesserung der Kollektionseffizienz des rückgestrahlten Lichtes als Polychromator mit relativ geringer spektraler Auslösung und weiten Spektralfenstern ausgeführt sein. Im letzteren Fall kann auch die Sammellinse vor dem Polychromator 11 ggf. entfallen.

Da nur Licht ausgewertet wird, dessen Photonen eine höhere Energie haben, als die des eingestrahlten Lichtes, ist sichergestellt, dass das Signal aus nichtlinearen Prozessen stammt, die nur im Bereich des Fokus stattfinden können. Aus der Position einer Linse 5 und dem Brechungsindex des Gewebes ist die Lage des Fokus 51 eindeutig bestimmt. Die Tiefenzuordnung kann an einem geeignet präparierten Objekt kalibriert werden.

Die Intensität wird während des Tiefenscans variiert, um die Abschwächung des Anregungslichtes bei der Fokussierung in tiefere Bereiche des Gewebes auszugleichen. Die Regelung basiert auf physikalischen Effekten, die gewebeunabhängig als Funktion der Anregungsintensität im Fokus ausgelöst werden und gleichen somit auch die Abschwächung in unterschiedlichen Gewebetypen aus. Auf diese Weise werden störende Selbstfokussierung sowie quantitative UV-Konvertierung im Gewebe vermieden.

In Fig. 4A und 4B ist ohne Beschränkung der Allgemeinheit die Relativbewegung zwischen Strahl und Fokus jeweils nur in zwei Koordinaten (x lateral, z in die Gewebetiefe) dargestellt. Die zweite Lateralachse y kann erfindungsgemäß in einer der angegebenen Weisen zusätzlich abgefahren werden.

Das Prinzip der Anordnung der Fig. 4B entspricht dem der Fig. 4A, mit dem Unterschied, dass die Probe mit einer Positioniereinheit 12 gegenüber dem fokussierenden Objektiv bewegt werden kann, um verschiedene Punkte der Probe ausmessen zu können. Des Weiteren ist ein Spektrometer oder Polychromator (siehe entsprechende Bemerkung zu Fig. 4A) 11a angeordnet, das zeitaufgelöste Messungen ermöglicht und mit einer Auswerteeinheit 13 verbunden ist. Die Anordnung nach Fig. 4B ist besonders geeignet für invitro-Untersuchungen, wie z. B. Zellkulturen, von exzidierten Gewebeproben oder pathologischen Präparaten.

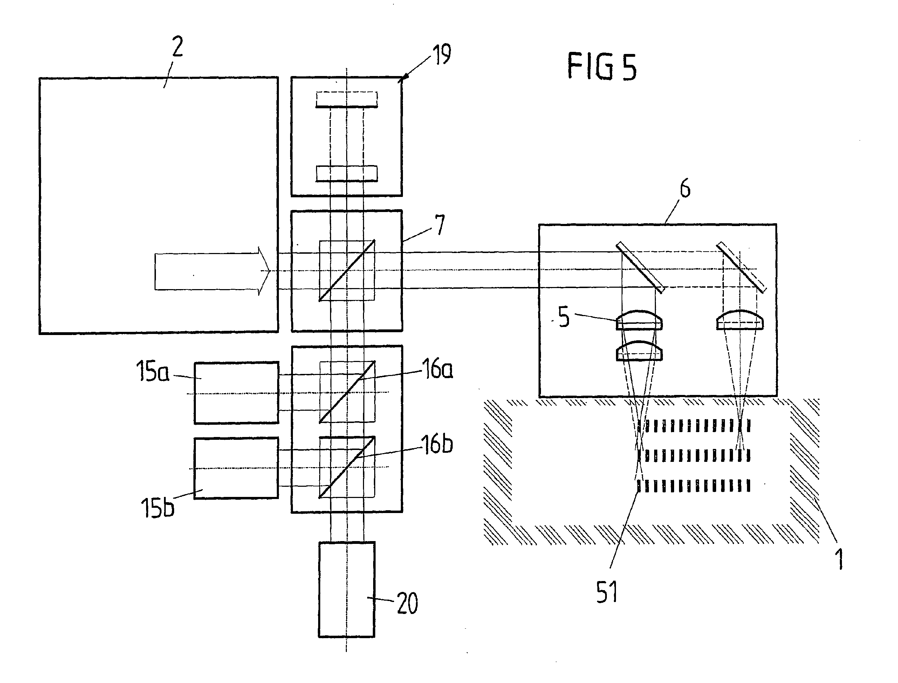

Die Fig. 5 zeigt eine weitere Variante einer Apparatur zur optischen Gewebeuntersuchung mit kombinierten Fluoreszenz- und OCT-System. Das Prinzip dieses Systems ist bereits in Bezug auf die Fig. 3 erläutert worden. Die Apparatur ist nicht mit einem Spektrometer ausgestattet, sondern wird mit mehreren diskreten Sensoren 15a, 15b betrieben, die durch Filterung mittels vorgeschalteter Filter 16a, 16b spektrale Teilsignale des Fluoreszenzspektrums nutzen.

Bei den Filtern 16a, 16b handelt es sich nicht ausschließlich um einfach Linien-, Band- oder Kantenfilter. Vielmehr werden mindestens teilweise komplexe Verlaufsfilter eingesetzt werden, die gemäß einer Bewertungsfunktion berechnet und hergestellt sind.

Beispielhaft seien im Folgenden einige typische Betriebswerte einer derartigen Apparatur tabellarisch dargestellt. In Übertragung der Ergebnisse aus den Vorversuchen lässt sich mit folgenden Geräteparametern eine Bestimmung der angestrebten spezifischen Messergebnisse für die Auswertung in einer Schrittweite von 50 $\mu$m lateral wie saggital und einer Bildwiederholrate von 2 Hz auf einem Schnitt von 1.5 x 4 mm$^2$ erreichen:

| Bezeichnung | | Wert | | Bemerkung |
|---|---|---|---|---|
| **Laserdaten:** | | | | |
| Mittlere Leistung | **P** | 90 | mW | |
| Pulsdauer | $\tau$ | 10 | ps | |
| Pulswiederholfrequenz | $f_{rep}$ | 50 | kHz | |
| **Strahldaten** | | | | |
| Strahldurchmesser im Fokus | $\mathbf{D_{fok}}$ | 10 | $\mu$m | |
| Rayleigh-Länge | $\mathbf{Z_{fok}}$ | 80 | $\mu$m | = "Schärfentiefe" |
| Wirkvolumen | $\mathbf{V_{fok}}$ | 12.000 | $(\mu m)^3$ | = $\pi/4 * D_{fok}^2 * 2 Z_{fok}$ |
| Intensität im Fokus | | $10^{11}$ | W/cm$^2$ | Spitzenleistung im Pulsmaximum, durch Intensitätsregelung festgelegt |
| **Scandaten** | | | | |
| Scanfrequenz | $\mathbf{f_{scan}}$ | 5 | kHz | Mittelung über 10 Laserpulse, die kontinuierlich im Gewebe bewegt werden. |
| Scanschritt lateral | $\Delta\mathbf{x}, \Delta\mathbf{y}$ | 50 | $\mu$m | = parallel zur Hautoberfläche |
| Scanschritt axial | $\Delta\mathbf{z}$ | 50 | $\mu$m | = in die Gewebetiefe |

Die Apparatur gemäß der Fig. 5 arbeitet mit nicht-linearer Fluoreszenzanregung. Dies aus folgenden Gründen:

A. Eine ausreichende Eindringtiefe ist nur innerhalb des "IR-Fensters" von ca. 750 bis 900 nm möglich.

B. Durch die nichtlineare Wirkung geschieht die Fluoreszenzanregung praktisch ausschließlich im Focus der Anregung, daher sehr lokalisiert, so dass eine hohe laterale und Tiefenauflösung erreicht wird.

C. Die durch nicht-lineare Anregung gewonnenen Fluoreszenzspektren unterscheiden sich nach den bisherigen eigenen Messungen grundsätzlich von solchen, die mittels linearer Anregung , d. h. mit der halben Anregungs-Wellenlänge, gewonnen werden.

Die Ursache liegt darin, dass für den Fluoreszenzvorgang, sowohl die Anregungskinetik, als auch die Abstrahlungskinetik sowie intra-molekulare Energieübertragungsvorgänge wesentlich sind. Diese Erkenntnis ist aus einschlägigen wissenschaftlichen Untersuchungen grundsätzlich bekannt. Die damit verbundene wesentlich verbesserte Spezifität von nichtlinearen laserspektroskopischen Methoden wird inzwischen in einigen technischen und biologisch-medizinischen Applikationen fruchtbringend angewendet.

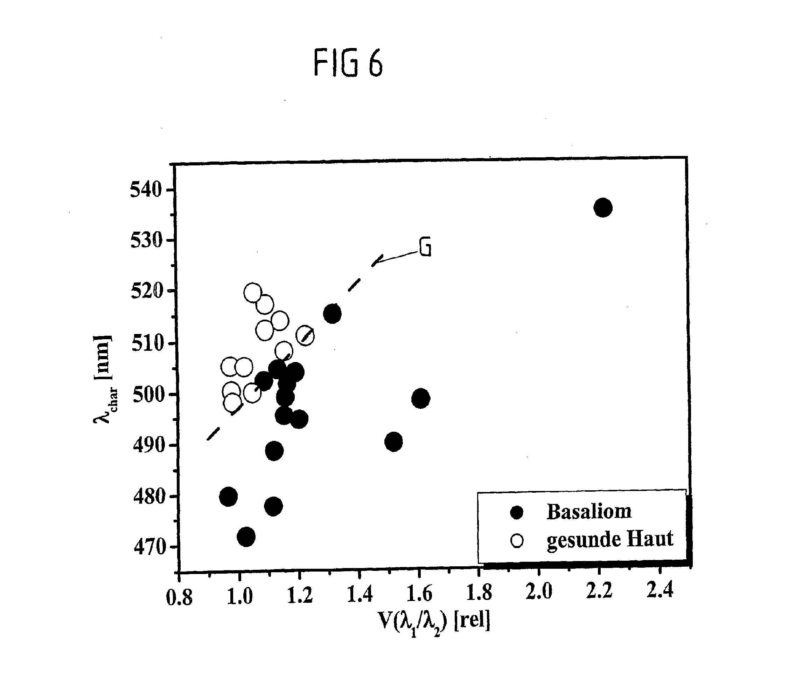

Im Rahmen von Voruntersuchungen zu dem genannten Punkt C werden Proben herangezogen und in vitro untersucht, die histopathologisch definiert waren (Basaliom/ Haut). Der neue Ansatz - nichtlineare Spektroskopie in suprazellulärer Mittelwertmessung - hat in den Untersuchungen eine sehr gute Unterscheidbarkeit zwischen Basaliom und normalem Gewebe ermöglicht. Es konnten in den so erzeugten Emissionsspektren Spektralbereiche identifiziert werden, die praktisch eine Ja/Nein-Entscheidung bezüglich normalem oder anomalem Zellstoffwechsel erlauben. In einer Vorstudie wurden mit diesem Verfahren inzwischen insgesamt 26 Messreihen durchgeführt und ausgewertet. Das Ergebnis ist in der Fig. 6 dargestellt.

Die Fig. 6 zeigt eine Auswertung der nichtlinear angeregten Emissionsmessungen an 26 Proben, von denen 11 gesundes und 15 krankes Gewebe darstellen. In der Ordinate ist die Lage einer aus dem Spektrum berechneten charakteristischen Wellenlänge ($\lambda_{char}$) und in der Abszisse das Intensitätsverhältnis an zwei festgelegten Wellenlängen $V(\lambda_1/\lambda_2)$ aufgetragen. Die unterbrochene Linie G stellt eine Grenzkurve dar. Es ist erkennbar, dass Basaliome von normaler Haut unterschieden werden können.

Es zeigt sich, dass durch geeignete mathematische Reduzierung der Messergebnisse eine selbstreferenzierte, d.h. von der Gesamtintensität unabhängige, skalare Kenngröße ermittelt werden kann, die im Folgenden als Fluoreszenzoptischer Gewebs Indikator (FGI) bezeichnet werden soll. Der FGI zu einem bestimmten geometrischen Messpunkt im Gewebe kann aus den Intensitätswerten bei einigen charakteristischen Wellenlängen ermittelt werden. Die Auswertung dieser Messungen zeigt, dass es in nahezu eindeutiger Weise möglich ist, krankes von gesundem Gewebe mit dieser nichtlinearen Methode zu unterscheiden. Das genaue Verfahren ist Gegenstand der Patentanmeldung.

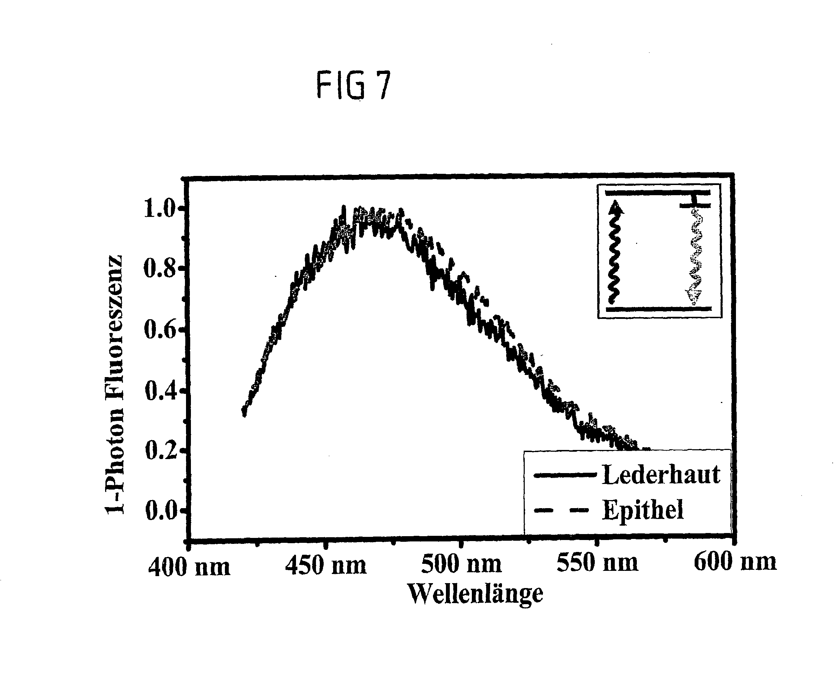

Hintergrund für diese neue Methode ist die Tatsache, dass im kranken Gewebe eine Reihe von molekularen Indikatoren vorliegen. Diese können jedoch auf Grund ihrer spektroskopischen Eigenschaften mit linearen Methoden nicht unterschieden werden, wie die Fig. 7 zeigt.

Die Fig. 7 zeigt eine konventionelle fluoreszenzspektroskopische Untersuchung (UVinduzierte Autofluoreszenz mit Einphotonen-Anregung)) von unterschiedlichen Gewebetypen (Lederhaut mit durchgezogener Linie bzw. Epithelgewebe mit gestrichelter Linie dargestellt). Aus dem Spektrum ist erkennbar, dass keine signifikanten spektroskopischen Unterschiede existieren. Dieses ist nicht verwunderlich, da die in diesem Zusammenhang betrachteten Substanzen sich in ihrem molekularen Aufbau nur sehr wenig unterscheiden. Erfahrungsgemäß ist durch die breiten Absorptionsbanden und die statistische Mittelung dann auch keine Spezifität zu erwarten.

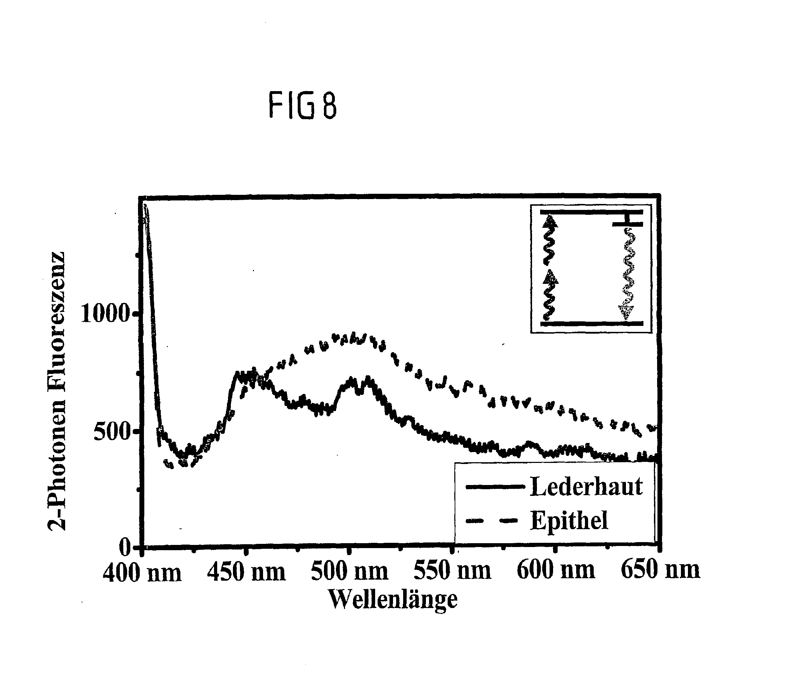

Eine nichtlineare Methode schafft hier Abhilfe, indem die unter nichtlinearen Bedingungen deutlich verstärkten Unterschiede zwischen den verschiedenen molekularen Systemen spektroskopisch auswertbar werden. Wie groß diese Unterschiede sind, ist in der Fig. 8 dargestellt. Die Fig. 8 zeigt das Ergebnis einer nichtlinear angeregten spektroskopische Untersuchung der unterschiedlichen Gewebetypen. Im Gegensatz zu Abb. 7 existieren signifikante Unterschiede.

Diese wenigen Beispiele sollen belegen, dass die nichtlinearen spektroskopischen Methoden einen grundsätzlich neuen Zugang zur Analytik gerade im biologisch-medizinischen Bereich eröffnen. Diese speziellen Beispiele zeigen ohne Einschränkung der Allgemeinheit die Prinzipien der neuartigen Methodik und belegen ihre Realisierbarkeit. Der erfindungsgemäß breitere Ansatz ist in den dargestellte Ansprüchen und Erläuterungen dargestellt.

Ein wesentlicher Teil des Ansatzes des erfindungsgemäßen Verfahrens ist, dass der FGI mit einer räumlichen Auflösung detektiert wird, die über das Volumen (Fokussierungsvolumen) von 20 bis 200.000 Zellen mittelt. Es handelt sich beim fluoreszenzoptischen Gewebsindikator also um eine suprazellulär gemittelte Größe, die das Gewebe beschreibt, und nicht um eine Kenngröße für eine Zellorganelle oder bestimmte Zellkompartimente. Erst diese Festlegung ermöglicht durch Datenreduktion auf einen dem Diagnosezweck angemessenen Umfang eine zeitliche Auswertung in Echtzeit, ohne Fixierung des Patienten über lange Zeiträume.

Die Auftragung des FGI für die bis jetzt durchgeführten Gewebsmessungen gibt eine klare Abgrenzung zwischen gesundem und krankem Gewebe (siehe Abb. 4). Zusammenfassend lässt sich feststellen, dass Selektivität durch die Auswertung der Fluoreszenzdaten erzielt wird. Die zeitlich und örtlich deckungsgleiche Kombination dieser Fluoreszenzdaten mit einem OCT erlaubt die Orientierung des Arztes im Gewebe und liefert die Zuordnung der fluoreszenzoptischen Messungen zur Morphologie des untersuchten Hautareals.

Die bisherigen Untersuchungen zeigen, dass mit dem Fluoreszenzgewebeindikator FGI in Verbindung mit charakteristischen Fluoreszenzmaxima bereits bei reiner Intensitätsauswertung und Verhältnisbildung bei zwei Wellenlängen (selbstreferenziert) ein klarer Indikator für pathologische Zustände des Gewebes gefunden werden konnte.

Mit der beispielhaftdargelegten Definition des FGI wurde also bereits jetzt ein klar abzugrenzender Indikatorwert gefunden, der durch Schwellwerte eine automatische Unterscheidung zwischen gesund und krank ermöglicht. Die dargestellten erfindungsgemäßen Verfahrensgrundlagen erlauben es, die Selektivität und Spezifität weiter zu erhöhen und an vielfältige Fragestellungen spezifisch anzupassen.

So werden erfindungsgemäß zur Erreichung eines optimalen und möglichst aussagekräftigen Diagnoseziels, mit minimalen falsch-negativen und falsch-positiven Aussagen und zur Erhöhung der Diagnoseaussage, wie dargelegt, andere Wellenlängen herangezogen und/oder ein Korrelationsfaktor aus mehr als zwei Wellenlängen berechnet.

Es können aus dem Bildpunkt bzw. Fokuspunkt die Intensitätswerte der Fluoreszenzstrahlung bei zwei oder mehr signifikanten Indikator- bzw. Referenz-Wellenlängen bestimmt und ins Verhältnis gesetzt werden. Damit lässt sich die gewünschte Geschwindigkeit der Auswertung erzielen und die Möglichkeit gewinnen, in Echtzeit sowohl die Mess-Signale auszuwerten, als auch die Gut-/ Schlechtentscheidung zu treffen, sowie das Ergebnis im OCT-Bild darzustellen.

Dabei kann zusätzlich

a) das Abklingverhalten, d. h. die zeitliche Komponente der Fluoreszenzabstrahlung, sowie
b) der Einfluss der Fluoreszenz-Anregungswellenlänge berücksichtigt werden.

Zu a:    Mittels geeigneter schneller Sensoren mit festgelegter Detektionswellenlänge (beispielsweise PMT = Photo Multiplier Tubes) kann der zeitliche Verlauf durch elektronisches Gating ausgewertet werden.

Zu b:    Durch eine Variation der Anregungswellenlänge wird die Detektions-Sicherheit und -Schärfe erfindungsgemäß erhöht.

Optionale zusätzliche Verfahren erlauben in ihrer Kombination mit der beschriebenen neuen Methode die Gewinnung zusätzlicher, für den Mediziner nützlicher, Informationen.

Zum einen handelt es sich dabei um die mögliche Kombination mit einem OCT-Verfahren, welches für die Spezifität des beantragten neuen Verfahrens nicht erforderlich ist, dem Mediziner jedoch morphologische Zusatzinformationen gibt, die ihm die weitere Behandlung erleichtern.

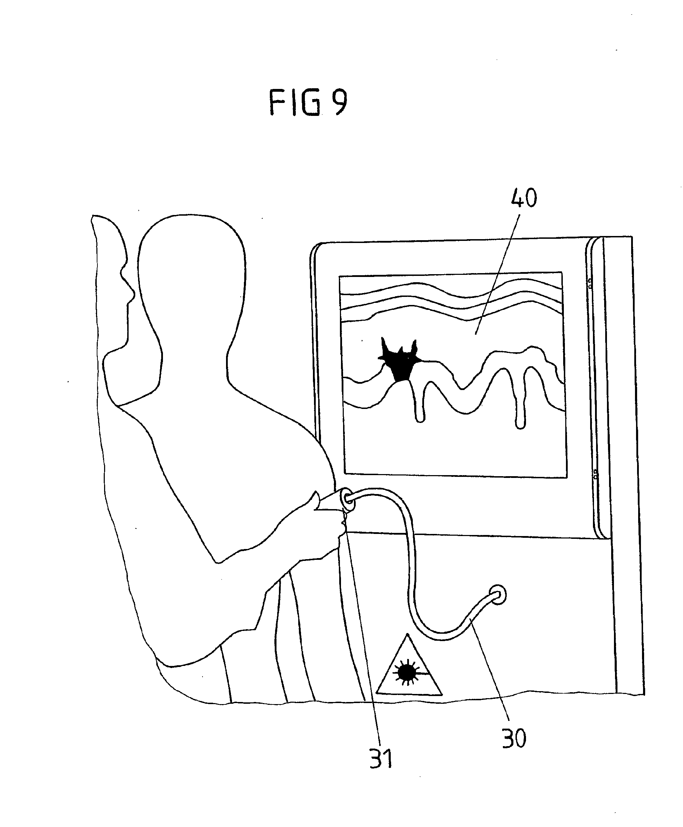

In Fig. 9 ist eine weitere Ausführungsform einer Vorrichtung zur optischen Gewebeuntersuchung gezeigt. Eine Laserquelle (nicht dargestellt) ist über eine Glasfaser 30 mit einem Messkopf 31 verbunden. Über die Glasfaser 30 und der Messkopf 31 kann das Laserlicht an das zu untersuchende Gewebe heran- und hinweggeführt werden. Somit kann ähnlich einer Ultraschalluntersuchung ein weites Gewebeareal optisch "abgetastet" werden. Das rückgestreute bzw. im Gewebe angeregte Licht wird über die Glasfaser 30 in eine integrierte Auswerteeinheit geleitet, die aus den bestimmten Daten eine Bildinformation über das Gewebe generiert. Diese Bildinformation wird an einem integrierten Bildschirm 40 angezeigt.

Zusätzlich zu einem Fluoreszenzsystem kann ein OCT-System integriert sein, das synchron und örtlich deckungsgleich mit dem Fluoreszenzsystem arbeitet.

Dies lässt sich mit relativ geringem apparativem Aufwand realisieren, da die Optik, der Scanner, die Laserquelle und der Signalweg des Fluoreszenzsystems genutzt werden können.

Dieses OCT liefert als wichtige Zusatzinformation die morphologische Struktur derjenigen Gewebevolumina, für die die genannten Informationen zu abweichendem Stoffwechsel fluoreszenzoptisch bestimmt werden. Damit kann diese Information unmittelbar dem morphologischen Gewebebild zugeordnet werden.

Aus der geometrischen Zuordnung zum Messkopf wird so ein Bild erzeugt, das auf Gewebsstrukturen bezogen ist. Der Arzt erhält eine Aussage in welchen gewebsmorphologisch charakterisierten Bereichen im Gewebe die kritischen Areale mit auffälligem Stoffwechsel liegen.

Kennzeichen der Apparatur ist ein für den Praxis- bzw. Klinikalltag taugliches Medizingerät zur effizienten, sicheren und reproduzierbaren Tumordiagnostik. Die Wirkprinzipien, Methoden und Algorithmen, die dazu verwendet werden, sind durch die medizinischdiagnostische Zielsetzung determiniert. Dies betrifft beispielsweise die Fragestellungen von Ein- oder Mehrfarbenanregung und der spektralen und zeitlich aufgelösten Detektion der Fluoreszenz. Aus der klinischen Praxis folgen die Dimensionen des diagnostischen Beobachtungsfeldes: eine Tiefenauflösung von 0,5 - 1,5 mm, Schnittbilddarstellungen mit mehreren Millimetern Kantenlänge (z. B. 4 mm).

Wesentliches Kennzeichen für die diagnostische Interpretation ist dabei die Zusammenführung der fluoreszenzoptischen Daten mit morphologischen Informationen, denn nur dadurch wird beispielsweise die kanzerogene Durchdringung der Basalmembran erkennbar. Für derartige morphologische Informationen wird erfindungsgemäß das Verfahren des OCT oder die Extraktion entsprechender Fluoreszenzsignale eingesetzt.

**Patentansprüche**

1. Verfahren zur optischen Charakterisierung von aus Zellen gebildetem menschlichem oder tierischem Gewebe mit den Schritten:

- Bereitstellen einer Strahlquelle zum Aussenden gerichteter elektromagnetischer Strahlung;
- Bestrahlen des zu charakterisierenden Gewebes mit der Strahlung, wodurch im Gewebe eine für das Gewebe charakteristische Rückstrahlung erzeugt wird, wobei
- die in das Gewebe eingedrungene Strahlung innerhalb eines Anregungsbereiches eine ausreichende Intensität aufweist, um im Gewebe eine charakteristische Rückstrahlung anzuregen, wobei

- der von der Strahlquelle ausgesandten Strahlung ein derartiges Intensitätsprofil quer zu ihrer Ausbreitungsrichtung aufgeprägt wird, dass der Anregungsbereich eine Mehrzahl von Zellen des Gewebes überdeckt und die angeregte Rückstrahlung auf zellübergreifende Gewebeeigenschaften zurückgeht, wobei
- das Aufprägen des Intensitätsprofils dadurch erfolgt, dass die von der Strahlquelle ausgesandte Strahlung in mindestens zwei Teilstrahlen aufgeteilt wird, wobei der Anregungsbereich durch die Lage der Teilstrahlen festgelegt ist, und wobei
- die Teilstrahlen jeweils so in das Gewebe fokussiert werden, dass die Fokuspunkte für die Teilstrahlen beabstandet zueinander sind,

**dadurch gekennzeichnet, dass**
die Rückstrahlung summarisch aus den Fokalbereichen aller Teilstrahlen erfasst wird.

2.  Verfahren nach Anspruch 1, **dadurch gekennzeichnet, dass** das Intensitätsprofil so gewählt wird, dass sich der Anregungsbereich eines einzelnen Teilstrahls quer zur Ausbreitungsrichtung der von der Strahlquelle ausgehenden Strahlung über näherungsweise 1 bis 2.500 Zellen des Gewebes erstreckt.

3.  Verfahren nach Anspruch 1 oder 2, **dadurch gekennzeichnet, dass** sich der Summenanregungsbereich der erzeugten Teilstrahlen quer zur Ausbreitungsrichtung der von der Strahlquelle ausgehenden Strahlung über näherungsweise 1.000 bis 200.000 Zellen des Gewebes erstreckt.

4.  Verfahren nach einem der vorhergehenden Ansprüche, **dadurch gekennzeichnet, dass** die Rückstrahlung ein Fluoreszenzsignal, eine harmonische Oberwelle der Strahlung der Strahlquelle und/ oder ein Reflexionssignal ist.

5.  Verfahren nach einem der vorhergehenden Ansprüche, **dadurch gekennzeichnet, dass** die Intensität der Strahlung so gewählt wird, dass eine Zwei- oder Mehrphotonenanregung im Gewebe erfolgt.

6.  Verfahren nach einem der vorhergehenden Ansprüche, **dadurch gekennzeichnet, dass** die von der Strahlquelle ausgesandte Strahlung zur zusätzlichen Charakterisierung des Gewebes per Kohärenz-Reflektometrie in einen Anregungsstrahl zur Anregung einer Fluoreszenz in dem Gewebe und einen Referenzstrahl aufgeteilt wird, wobei der Anregungsstrahl zum Teil vom Gewebe zurückreflektiert und der Referenzstrahl mit der vom Gewebe zurückreflektierten Strahlung überlagert wird.

7.  Verfahren nach Anspruch 6, **dadurch gekennzeichnet, dass** der Referenz- und der Anregungsstrahl in einem ortsauflösenden Interferenzdetektor überlagert werden.

8.  Verfahren nach Anspruch 7, **dadurch gekennzeichnet, dass** der Interferenzdetektor ein ein- oder zweidimensionaler Bildgeber mit mindestens 4 Pixeln, insbesondere ein CCD-Chip, eine CCD-Kamera, eine CCD-Zeile oder eine Zeilenkamera, ist.

9.  Verfahren nach einem der Ansprüche 6 bis 8, **dadurch gekennzeichnet, dass** das der von der Strahlquelle ausgehenden Strahlung aufgeprägte Intensitätsprofil einen charakteristischen Verlauf mit mindestens zwei Intensitätsmaxima aufweist.

10. Verfahren nach Anspruch 6, 7 und 9, **dadurch gekennzeichnet, dass** die optische Weglänge für den Referenzstrahl so eingestellt wird, dass sich die auf die von der Strahlquelle ausgesandten Strahlung aufgeprägte Intensitätsverteilung im Interferenzdetektor nachweisen lässt, wobei in diesem Fall die optische Weglänge für den Referenzstrahl der vom Anregungsstrahl zurückgelegten optischen Weglänge von der Strahlquelle bis zum Fokus und zurück bis zum Interferenzdetektor entspricht.

11. Verfahren nach Anspruch 10, **dadurch gekennzeichnet, dass**

a) der Interferenzdetektor die Intensität der an ihm eintreffenden Strahlung ortsaufgelöst in einer Ebene senkrecht zur Strahlung bestimmt;
b) jeweils die Intensitäten an benachbarten Orten miteinander vergleicht, um einen ortsabhängigen Kontrastwert zu ermitteln;
c) aus den ortsabhängigen Kontrastwerten einen Mittelwert bestimmt, der eine charakteristische, integrale Kontrastgröße für die am Detektor eintreffende Strahlung darstellt;
d) das aufgeprägte Intensitätsprofil dadurch nachgewiesen wird, dass die integrale Kontrastgröße ein Maximum erreicht.

12. Verfahren nach Anspruch 10, **dadurch gekennzeichnet, dass**

a) der Interferenzdetektor die Intensität der an ihm eintreffenden Strahlung ortsaufgelöst in einer Erfassungsebene senkrecht zur Strahlung bestimmt;
b) aus den bestimmten Intensitätswerten und dem auf die Strahlung der Strahlquelle aufgeprägten Intensitätsprofil einen Korrelationswert bestimmt wird;
c) das aufgeprägte Intensitätsprofil dadurch nachgewiesen wird, dass der Korrelationswert ein Maximum erreicht.

13. Verfahren nach einem der vorhergehenden Ansprüche, **dadurch gekennzeichnet, dass** die von der Strahlquelle ausgesandte Strahlung quer zu ihrer Ausbreitungsrichtung so abgelenkt wird, so dass ein Bereich des Gewebes, der sich parallel zur Gewebeoberfläche erstreckt, abgetastet wird.

14. Verfahren nach einem der vorhergehenden Ansprüche, **dadurch gekennzeichnet, dass** die Fokussierung der von der Strahlquelle ausgesandte Strahlung in eine Fokusebene zeitlich abhängig erfolgt, so dass die Fokusebene in Ausbreitungsrichtung der Strahlung verschoben wird, wodurch ein Bereich, der sich senkrecht zur Gewebeoberfläche erstreckt, abgetastet wird.

15. Verfahren nach einem der vorhergehenden Ansprüche, **dadurch gekennzeichnet, dass** die im Gewebe angeregte Rückstrahlung in einem Detektor (8) nachgewiesen wird, wobei ihre Intensität in Abhängigkeit von der Wellenlänge und/oder der Zeit bestimmt wird.

16. Verfahren nach einem der vorhergehenden Ansprüche, **dadurch gekennzeichnet, dass**

a) das Bestrahlen des Gewebes mit Strahlung im Wellenlängenbereich von 720-800 nm erfolgt, wodurch im Gewebe eine Zweiphotonen-Fluoreszenz angeregt wird;
b) Detektieren der Intensität eines ersten Fluoreszenzsignals bei einer Wellenlänge von $460 \pm 30$ nm und eines zweiten Fluoreszenzsignals bei einer Wellenlänge von $550 \pm 30$ nm;
a) Bestimmten des Verhältnisses der Intensitäten des ersten und zweiten Fluoreszenzsignals; und
b) in Abhängigkeit von dem in Schritt c) bestimmten Verhältnis ein Signal erzeugt wird.

17. Verfahren nach einem der vorhergehenden Ansprüche, **dadurch gekennzeichnet, dass**

a) das Gewebe mit Strahlung im Wellenlängenbereich zwischen 500-550 nm bestrahlt wird, und dabei
b) eine erste Intensität einer Zweiphotonen-Fluoreszenz bei einer Wellenlänge von $340 \pm 40$ nm detektiert wird;
c) das Gewebe mit Strahlung im Wellenlängenbereich zwischen 720-800 nm bestrahlt wird, und dabei
d) eine zweite Intensität einer Zweiphotonen-Fluoreszenz bei einer Wellenlänge von $460 \pm 40$ nm detektiert wird;
e) das Verhältnis der in Schritt b) bestimmten ersten Intensität und der in Schritt d) bestimmten zweiten Intensität bestimmt wird; und
f) in Abhängigkeit von dem in Schritt e) bestimmten Verhältnis ein Signal erzeugt wird.

18. Verfahren nach einem der vorhergehenden Ansprüche, **dadurch gekennzeichnet, dass**

a) das Gewebe mit Strahlung im Wellenlängenbereich zwischen 720-800 nm bestrahlt wird, und dabei
b) eine Intensität einer Zweiphotonen-Fluoreszenz bei einer Wellenlänge von $460 \pm 40$ nm detektiert wird.

19. Verfahren nach Anspruch 18, **dadurch gekennzeichnet, dass** die Strahlung über ein Objektiv in das Gewebe abgebildet wird und das Objektiv entlang der Ausbreitungsrichtung der von der Strahlquelle ausgesandten Strahlung schrittweise verschoben wird, wobei die Schritte a) und b) für mehrere Objektivpositionen erfolgen, so dass der Fokus in Ausbreitungsrichtung der Strahlung durch das Gewebe geführt und ein Fluoreszenzsignal für mehrere Gewebetiefen bestimmt wird.

20. Verfahren nach Anspruch 19, **dadurch gekennzeichnet, dass** das Verfahren an einem gesunden Gewebe durchgeführt wird und die so bestimmten Intensitäten des Fluoreszenzsignals als Referenzwerte gespeichert werden.

21. Verfahren nach Anspruch 19 und 20, **dadurch gekennzeichnet, dass**

a) das Verfahren gemäß Anspruch 19 an einem zu charakterisierenden Gewebe durchgeführt wird,

b) die bestimmten Intensitäten für jede Tiefe zu den jeweiligen Intensitäten des gespeicherten Referenzsignals ins Verhältnis gesetzt werden;

c) in Abhängigkeit von dem in Schritt b) bestimmten Verhältnis ein Signal erzeugt wird.

22. Verfahren nach Anspruch 21, **dadurch gekennzeichnet, dass** das Signal erzeugt wird, wenn das in Schritt b) bestimmte Verhältnis näherungsweise 30% von dem Wert 1 abweicht.

23. Verfahren nach einem der vorhergehenden Ansprüche, **dadurch gekennzeichnet, dass**

a) das Bestrahlen des Gewebes mit einem Kurzpulslaserstrahl im Wellenlängenbereich 720-880 nm oder 980-1080 nm erfolgt;

b) Detektieren der Intensität remittierender Strahlung bei der halben Wellenlänge der gemäß Schritt a) zum Bestrahlen des Gewebes verwendeten Strahlung.

24. Verfahren nach Anspruch 23, **dadurch gekennzeichnet, dass** die Strahlung über ein Objektiv in das Gewebe abgebildet wird und das Objektiv entlang der Ausbreitungsrichtung der von der Strahlquelle ausgesandten Strahlung schrittweise verschoben wird, wobei die Schritte a) und b) für mehrere Objektivpositionen erfolgen, so dass der Fokus in Ausbreitungsrichtung der Strahlung durch das Gewebe geführt und ein Fluoreszenzsignal für mehrere Gewebetiefen bestimmt wird.

25. Verfahren nach Anspruch 24, **dadurch gekennzeichnet, dass** das Verfahren an einem gesunden Gewebe durchgeführt wird und die so bestimmten Intensitäten des Fluoreszenzsignals als Referenzwerte gespeichert werden.

26. Verfahren nach Anspruch 24 und 25, **dadurch gekennzeichnet, dass**

a) das Verfahren gemäß Anspruch 23 an einem zu charakterisierenden Gewebe durchgeführt wird;

b) die bestimmten Intensitäten für jede Tiefe zu den jeweiligen Intensitäten des gespeicherten Referenzsignals ins Verhältnis gesetzt werden;

c) in Abhängigkeit von dem in Schritt b) bestimmten Verhältnis ein Signal erzeugt wird.

27. Verfahren nach Anspruch 26, **dadurch gekennzeichnet, dass** das Signal erzeugt wird, wenn das in Schritt b) bestimmte Verhältnis näherungsweise 40% von dem Wert 1 abweicht.

28. Vorrichtung zur Durchführung eines Verfahrens nach einem der vorhergehenden Ansprüche, mit:

- einer Strahlquelle (2) zum Aussenden gerichteter Strahlung zum Bestrahlen des zu charakterisierenden Gewebes (1), die konfiguriert ist, im Gewebe eine für das Gewebe charakteristische Rückstrahlung anzuregen, wobei

- in das Gewebe eingedrungene Strahlung innerhalb eines sich quer zu ihrer Ausbreitungsrichtung erstreckenden Anregungsbereiches eine ausreichende Intensität aufweist, um im Gewebe eine charakteristische Rückstrahlung anzuregen, und

- Strahlformungsmitteln (3), die konfiguriert sind, der von der Strahlquelle (2) ausgesandten Strahlung ein derartiges Intensitätsprofil aufzuprägen, dass der Anregungsbereich eine Mehrzahl von Zellen des Gewebes überdeckt und die beim Bestrahlen des Gewebes angeregte Rückstrahlung auf zellübergreifende Gewebeeigenschaften zurückgeht, wobei

- die Strahlformungsmittel (3) derart ausgebildet sind, die von der Strahlquelle ausgesandte Strahlung in mindestens zwei Teilstrahlen (4a,4b,4c) aufzuteilen, wobei der Anregungsbereich durch die Lage der Teilstrahlen festgelegt ist, wobei

- Abbildungsmittel (5,6) vorhanden sind, die derart ausgebildet sind, die Teilstrahlen (4a,4b,4c) jeweils so in das Gewebe (1) zu fokussieren, dass die Fokuspunkte (51a,51b,51c) für die Teilstrahlen beabstandet zueinander sind; und

- einem Detektor (8) zum Detektieren der Rückstrahlung, der in Abhängigkeit von der detektierten Strahlung ein elektrisches Signal erzeugt,

**dadurch gekennzeichnet, dass**
der Detektor (8) derart konfiguriert ist, die Rückstrahlung summarisch aus den Fokalbereichen aller in das Gewebe (1) fokussierten Teilstrahlen zu erfassen.

**29.** Vorrichtung nach Anspruch 28, **dadurch gekennzeichnet, dass** die Abbildungsmittel von der Strahlquelle aus gesehen vor oder hinter den Strahlformungsmitteln angeordnet sind.

**30.** Vorrichtung nach Anspruch 28, **dadurch gekennzeichnet, dass** die Abbildungsmittel mehrere Objektive umfassen und jeder Teilstrahl mit einem eigenen Objektiv in das Gewebe fokussiert wird.

**31.** Vorrichtung nach einem der Ansprüche 28 bis 30, **dadurch gekennzeichnet, dass** die Abbildungsmittel (5,6) mindestens ein Objektiv (5) zum Fokussieren der Strahlung sowie eine Verstelleinheit (6) umfassen, mit das mindestens eine Objektiv entlang der Ausbreitungsrichtung der Strahlung bewegt werden kann, um den Fokuspunkt des Objektivs entlang dieser Richtung zu bewegen und ein Abtasten des Gewebes in einer Ebene, die sich senkrecht zur Gewebeoberfläche erstreckt, zu ermöglichen.

**32.** Vorrichtung nach Anspruch 31, **dadurch gekennzeichnet, dass** die Verstelleinheit (6) und die Objektive in ein Gehäuse integriert sind, das auf das zu untersuchende Gewebe aufgelegt werden kann und parallel zur Gewebeoberfläche verschiebbar ist.

**33.** Vorrichtung nach Anspruch 32, **gekennzeichnet durch** elektromotorische und/oder mikromechanische Stellelemente, mit denen das Gehäuse parallel zur Gewebeoberfläche verschoben werden kann.

**34.** Vorrichtung nach einem der Ansprüche 28 bis 33, **gekennzeichnet durch** eine Lichtleitfaser (30), die die von der Strahlquelle erzeugte Strahlung an das zu charakterisierende Gewebe heranführt.

**35.** Vorrichtung nach Anspruch 34, **dadurch gekennzeichnet, dass** die Lichtleitfaser (30) die Abbildungsmittel und/oder die Strahlformungsmittel aufweist.

**36.** Vorrichtung nach Anspruch 35, **dadurch gekennzeichnet, dass** die Abbildungsmittel (5,6) und/oder die Strahlformungsmittel einstückig mit der Lichtleitfaser ausgebildet sind.

**37.** Vorrichtung nach einem der Ansprüche 28 bis 36, **gekennzeichnet durch** eine Auswerteeinheit zum Auswerten des vom Detektor (8) erzeugten elektrischen Signals.

**38.** Vorrichtung nach Anspruch 37, **dadurch gekennzeichnet, dass** die Auswerteinheit das vom Detektor (8) erzeugte elektrische Signal in ein Bildinformationssignal umsetzt.

**39.** Vorrichtung nach Anspruch 38, **dadurch gekennzeichnet, dass** die Auswerteeinheit einen Bildschirm (40) zur visuellen Darstellung des Bildinformationssignals aufweist.

**Claims**

**1.** Method for visual characterization of human or animal tissue formed from cells, comprising the following steps:

- providing a radiation source for emitting directional electromagnetic radiation;
- irradiating the tissue to be characterized with the radiation, whereby a reflection radiation which is characteristic of the tissue is generated in the tissue, wherein
- the radiation that has penetrated into the tissue having within an excitation region a sufficient intensity to excite a characteristic reflection radiation in the tissue, wherein
- the radiation emitted by the radiation source has impressed on it an intensity profile transversely with respect to its propagation direction, said intensity profile being such that the excitation region covers a plurality of cells of the tissue and the excited reflection radiation originates from inter-cell tissue properties, wherein
- impressing the intensity profile is effected by the radiation emitted by the radiation source being split into at least two partial beams, wherein the excitation region is defined by the position of the partial beams, and wherein
- the partial beams are in each case focused into the tissue in such a way that the focal points for the partial beams are spaced apart from one another,

**characterized in that**
the reflection radiation is detected summationally from the focal regions of all partial beams.

**2.** Method according to Claim 1, **characterized in that** the intensity profile is chosen in such a way that the excitation region of an individual partial beam, transversely with respect to the propagation direction of the radiation issuing from the radiation source, extends over approximately 1 to 2 500 cells of the tissue.

**3.** Method according to Claim 1 or 2, **characterized in that** the summation excitation region of the partial beams generated, transversely with respect to the propagation direction of the radiation issuing from the radiation source, extends over approximately 1 000 to 200 000 cells of the tissue.

**4.** Method according to one of the preceding clams, **characterized in that** the reflection radiation is a fluorescence signal, a harmonic of the radiation of the radiation source and/or a reflection signal.

**5.** Method according to one of the preceding claims, **characterized in that** the intensity of the radiation is chosen in such a way that two- or multiphoton excitation takes place in the tissue.

**6.** Method according to one of the preceding claims, **characterized in that** the radiation emitted by the radiation source, for additional characterization of the tissue by coherence reflectometry, is split into an excitation beam for exciting a fluorescence in the tissue and a reference beam, the excitation beam being partly reflected back from the tissue and the reference beam being superimposed with the radiation reflected back from the tissue.

**7.** Method according to Claim 6, **characterized in that** the reference and excitation beams are superimposed in a spatially resolving interference detector.

**8.** Method according to Claim 7, **characterized in that** the interference detector is a one- or two-dimensional image generator having at least 4 pixels, in particular a CCD chip, a CCD camera, a CCD line or a line camera.

**9.** Method according to one of Claims 6 to 8, **characterized in that** the intensity profile impressed on the radiation issuing from the radiation source has a characteristic variation having at least two intensity maxima.

**10.** Method according to Claims 6, 7 and 9, **characterized in that** the optical path length for the reference beam is set in such a way that the intensity distribution impressed on the radiation emitted by the radiation source can be detected in the interference detector, in this case the optical path length for the reference beam corresponding to the optical path length covered by the excitation beam from the radiation source to the focus and back to the interference detector.

**11.** Method according to Claim 10, **characterized in that**

a) the interference detector determines the intensity of the radiation arriving at it in a spatially resolved manner in a plane perpendicular to the radiation;
b) in each case compares the intensities at adjacent locations with one another in order to determine a location-dependent contrast value;
c) determines from the location-dependent contrast values an average value representing a characteristic integral contrast variable for the radiation arriving at the detector;
d) the impressed intensity profile is detected by the integral contrast variable attaining a maximum.

**12.** Method according to Claim 10, **characterized in that**

a) the interference detector determines the intensity of the radiation arriving at it in a spatially resolved manner in a detection plane perpendicular to the radiation;
b) a correlation value is determined from the intensity values determined and the intensity profile impressed onto the radiation of the radiation source;
c) the impressed intensity profile is detected by the correlation value attaining a maximum.

**13.** Method according to one of the preceding claims, **characterized in that** the radiation emitted by the radiation source is deflected transversely with respect to its propagation direction such that a region of the tissue extending parallel to the tissue surface is scanned.

**14.** Method according to one of the preceding claims, **characterized in that** the focusing of the radiation emitted by the radiation source into a focal plane is effected in a temporally dependent manner, such that the focal plane is displaced in the propagation direction of the radiation, whereby a region extending perpendicular to the tissue surface is scanned.

**15.** Method according to one of the preceding claims, **characterized in that** the reflection radiation excited in the tissue is detected in a detector (8), its intensity being determined in a manner dependent on the wavelength and/or the time.

**16.** Method according to one of the preceding claims, **characterized in that**

a) the tissue is irradiated with radiation in the wavelength range of 720-800 nm, whereby a two-photon fluorescence is excited in the tissue;
b) detection of the intensity of a first fluorescence signal at a wavelength of $460\pm30$ nm and of a second fluorescence signal at a wavelength of $550\pm30$ nm;
c) determination of the ratio of the intensities of the first and second fluorescence signals;
d) a signal is generated in a manner dependent on the ratio determined in step c).

**17.** Method according to one of the preceding claims, **characterized in that**

a) the tissue is irradiated with radiation in the wavelength range between 500-550 nm, and in this case
b) a first intensity of a two-photon fluorescence is detected at a wavelength of $340\pm40$ nm;
c) the tissue is irradiated with radiation in the wavelength range between 720-800 nm, and in this case
d) a second intensity of a two-photon fluorescence is detected at a wavelength of $460\pm40$ nm;
e) the ratio of the first intensity determined in step b) and the second intensity determined in step d) is determined; and
f) a signal is generated in a manner dependent on the ratio determined in step e).

**18.** Method according to one of the preceding claims, **characterized in that**

a) the tissue is irradiated with radiation in the wavelength range between 720-800 nm, and in this case
b) an intensity of a two-photon fluorescence is detected at a wavelength of $460\pm40$ nm.

**19.** Method according to Claim 18, **characterized in that** the radiation is imaged into the tissue via a lens and the lens is displaced step by step along the propagation direction of the radiation emitted by the radiation source, steps a) and b) being effected for a plurality of lens positions, such that the focus is led in the propagation direction of the radiation through the tissue and a fluorescence signal is determined for a plurality of tissue depths.

**20.** Method according to Claim 19, **characterized in that** the method is carried out on a healthy tissue and the intensities of the fluorescence signal that are thus determined are stored as reference values.

**21.** Method according to Claims 19 and 20, **characterized in that**

a) the method is carried out in accordance with Claim 19 on a tissue to be characterized,
b) the intensities determined for each depth are related to the respective intensities of the stored reference signal by a ratio;
c) a signal is generated in a manner dependent on the ratio determined in step b).

**22.** Method according to Claim 21, **characterized in that** the signal is generated if the ratio determined in step b) deviates approximately 30% from the value 1.

**23.** Method according to one of the preceding claims, **characterized in that**

a) the tissue is irradiated with a short-pulse laser beam in the wavelength range of 720-880 nm or 980-1080 nm;
b) detection of the intensity of returning radiation at half the wavelength of the radiation used for irradiating the .tissue in accordance with step a).

**24.** Method according to Claim 23, **characterized in that** the radiation is imaged into the tissue via a lens and the lens is displaced step by step along the propagation direction of the radiation emitted by the radiation source, steps a) and b) being effected for a plurality of lens positions, such that the focus is led in the propagation direction of the radiation through the tissue and a fluorescence signal is determined for a plurality of tissue depths.

25. Method according to Claim 24, **characterized in that** the method is carried out on a healthy tissue and the intensities of the fluorescence signal that are thus determined are stored as reference values.

26. Method according to Claims 24 and 25, **characterized in that**

a) the method is carried out in accordance with Claim 23 on a tissue to be characterized,
b) the intensities determined for each depth are related to the respective intensities of the stored reference signal by a ratio;
c) a signal is generated in a manner dependent on the ratio determined in step b).

27. Method according to Claim 26, **characterized in that** the signal is generated if the ratio determined in step b) deviates approximately 40% from the value 1.

28. Apparatus for carrying out a method according to one of the preceding claims , comprising:

- a radiation source (2) for emitting directional radiation for irradiating the tissue (1) to be characterized, which can excite in the tissue a reflection radiation which is characteristic of the tissue, wherein
- radiation that has penetrated into the tissue having, within an excitation region extending transversely with respect to the propagation direction of said radiation, a sufficient intensity to excite a characteristic reflection radiation in the tissue, and
- beam shaping means (3) with which the radiation emitted by the radiation source (2) can have impressed on it an intensity profile such that the excitation region can cover a plurality of cells of the tissue and the reflection radiation excited upon the irradiation of the tissue originates from inter-cell tissue properties, wherein
- the beam shaping means (3) are designed to split the radiation emitted by the radiation source into at least two partial beams (4a, 4b, 4c), wherein the excitation region is defined by the position of the partial beams, wherein
- imaging means (5, 6) are provided which are formed such that the partial beams (4a, 4b, 4c) in each case focused into the tissue (1) in such a way that the focal points (51a, 51b, 51c) for the partial beams are spaced apart from one another; and
- a detector (8) for detecting the reflection radiation, which generates an electrical signal in a manner dependent on the detected radiation,

**characterized in that**
the detector (8) is configured for summationally detection of the reflection radiation from the focal regions of all partial beams focused into the tissue (1).

29. Apparatus according to Claim 28, **characterized in that** the imaging means are arranged upstream or downstream of the beam shaping means as seen from the radiation source.

30. Apparatus according to Claim 28, **characterized in that** the imaging means comprise a plurality of lenses and each partial beam is focused into the tissue by a dedicated lens.

31. Apparatus according to one of Claims 28 to 30, **characterized in that** the imaging means (5, 6) comprise at least one lens (5) for focusing the radiation and also an adjusting unit (6), by which the at least one lens can be moved along the propagation direction of the radiation in order to move the focal point of the lens along said direction and to enable the tissue to be scanned in a plane extending perpendicular to the tissue surface.

32. Apparatus according to Claim 31, **characterized in that** the adjusting unit (6) and the lenses are integrated into a housing which can be placed onto the tissue to be examined and can be displaced parallel to the tissue surface.

33. Apparatus according to Claim 32, **characterized by** electromotive and/or micromechanical actuating elements by which the housing can be displaced parallel to the tissue surface.

34. Apparatus according to one of Claims 28 to 33, **characterized by** an optical fiber (30) that leads the radiation generated by the radiation source to the tissue to be characterized.

35. Apparatus according to Claim 34, **characterized in that** the optical fiber (30) has the imaging means and/or the beam shaping means.

36. Apparatus according to Claim 35, **characterized in that** the imaging means (5, 6) and/or the beam shaping means are formed integrally with the optical fiber.

37. Apparatus according to one of Claims 28 to 36, **characterized by** an evaluation unit for evaluating the electrical signal generated by the detector (8).

38. Apparatus according to Claim 37, **characterized in that** the evaluation unit converts the electrical signal generated by the detector (8) into an image information signal.

39. Apparatus according to Claim 38, **characterized in that** the evaluation unit has a screen (40) for visually displacing the image information signal.


**Revendications**

1. Procédé servant à la caractérisation optique de tissus humains ou animaux formés de cellules, comprenant les étapes suivantes :

   - la mise à disposition d'une source de rayonnement servant à émettre un rayonnement électromagnétique orienté ;
   - l'exposition du tissu à caractériser au rayonnement, ce qui permet de produire dans le tissu un renvoi du rayonnement caractéristique du tissu,
   - le rayonnement pénétrant le tissu présentant, dans une plage d'activation, une intensité suffisante pour activer, dans le tissu, un renvoi de rayonnement caractéristique,
   - le rayonnement émis par la source de rayonnement étant marqué, de manière transversale par rapport à son sens de propagation, d'un profil d'intensité tel que la zone d'activation couvre une pluralité de cellules du tissu et que le renvoi de rayonnement provoqué est lié aux propriétés du tissu transversales aux cellules,
   - la marquage du profil d'intensité étant réalisée de sorte que le rayonnement émis par la source de rayonnement est divisé en au moins deux faisceaux de rayons partiels, la zone d'activation étant définie par la position des faisceaux de rayons partiels, et
   - les faisceaux de rayons partiels étant respectivement concentrés dans le tissu de telle manière que les points de concentration des faisceaux de rayons partiels sont espacés les uns des autres,

   **caractérisé en ce que** le renvoi de rayonnement est détectée de manière globale à partir des zones de concentration de tous les faisceaux de rayons.

2. Procédé selon la revendication 1, **caractérisé en ce que** le profil d'intensité est choisi de telle sorte que la zone d'activation d'un faisceau de rayons partiel individuel s'étend de manière transversale par rapport au sens de propagation du rayonnement partant de la source de rayonnement sur approximativement 1 à 2.500 cellules du tissu.

3. Procédé selon la revendication 1 ou 2, **caractérisé en ce que** la zone d'activation globale des faisceaux de rayons partiels s'étend de manière transversale par rapport au sens de propagation du rayonnement partant de la source de rayonnement sur approximativement 1.000 à 200.000 cellules du tissu.

4. Procédé selon l'une quelconque des revendications précédentes, **caractérisé en ce que** le renvoi de rayonnement est un signal fluorescent, une harmonique du rayonnement de la source de rayonnement et/ou un signal de réflexion.

5. Procédé selon l'une quelconque des revendications précédentes, **caractérisé en ce que** l'intensité du rayonnement est choisie de manière à effectuer dans le tissu une activation biphotonique ou multiphotonique.

6. Procédé selon l'une quelconque des revendications précédentes, **caractérisé en ce que** le rayonnement émis par la source de rayonnement est divisé, aux fins de la caractérisation supplémentaire du tissu, par réflectométrie à cohérence, en un rayonnement d'activation servant à activer une fluorescence dans le tissu et en un rayonnement de référence, le rayonnement d'activation étant réfléchi en partie par le tissu et le rayonnement de référence étant recouvert d'un rayonnement réfléchi par le tissu.

7. Procédé selon la revendication 6, **caractérisé en ce que** le rayonnement de référence et le rayonnement d'activation sont superposés dans un détecteur d'interférence à résolution locale.

8. Procédé selon la revendication 7, **caractérisé en ce que** le détecteur d'interférence est un transmetteur d'image unidimensionnel ou bidimensionnel à au moins 4 pixels, en particulier une puce CCP, une caméra CCD, une ligne CCD ou une caméra linéaire.

9. Procédé selon l'une quelconque des revendications 6 à 8, **caractérisé en ce que** le profil d'intensité marqué au rayonnement partant de la source de rayonnement présente un tracé caractéristique doté d'au moins deux pics d'intensité.

10. Procédé selon la revendication 6, 7 et 9, **caractérisé en ce que** la longueur de trajet optique est réglée de telle manière pour le rayonnement de référence que la répartition d'intensité marquée sur le rayonnement émis par la source de rayonnement se laisse détecter dans le détecteur d'interférence, la longueur du trajet optique pour le rayonnement de référence correspondant dans le cas présent à la longueur de trajet optique parcourue par le rayonnement d'activation de la source de rayonnement jusqu'au point de concentration, puis jusqu'au détecteur d'interférence.

11. Procédé selon la revendication 10, **caractérisé en ce**

 a) **que** le détecteur d'interférence détermine l'intensité du rayonnement lui parvenant selon une résolution locale dans un plan perpendiculaire au rayonnement,
 b) en ce qu'il compare entre elles respectivement les intensités en des emplacements voisins afin de calculer une valeur de contraste dépendant de l'emplacement,
 c) en ce qu'il détermine, à partir des valeurs de contraste dépendant de l'emplacement, une valeur moyenne, qui illustre une grandeur de contraste intégrale caractéristique du rayonnement parvenant au détecteur,
 d) en ce que le profil d'intensité marqué est détecté lorsque la grandeur de contraste intégrale atteint un maximum.

12. Procédé selon la revendication 10, **caractérisé en ce**

 a) **que** le détecteur d'interférence détermine l'intensité du rayonnement lui parvenant, selon une résolution locale, dans un plan d'acquisition perpendiculaire par rapport au rayonnement,
 b) en ce qu'il détermine, à partir des valeurs d'intensité déterminées et du profil d'intensité marqué sur le rayonnement de la source de rayonnement, une valeur de corrélation,
 c) en ce que le profil d'intensité est détecté lorsque la valeur de corrélation atteint un maximum.

13. Procédé selon l'une quelconque des revendications précédentes, **caractérisé en ce que** le rayonnement émis par la source de rayonnement est dévié de manière transversale par rapport à son sens de propagation de telle manière qu'une zone du tissu, qui s'étend de manière parallèle par rapport à la surface du tissu, est balayée.

14. Procédé selon l'une quelconque des revendications précédentes, **caractérisé en ce que** la concentration du rayonnement émis par la source de rayonnement se fait dans un plan de concentration dépendant du temps de sorte que le plan de concentration est décalé dans le sens de propagation du rayonnement ce qui permet de balayer une zone qui s'étend de manière perpendiculaire par rapport à la surface du tissu.

15. Procédé selon l'une quelconque des revendications précédentes, **caractérisé en ce que** le renvoi de rayonnement activé dans le tissu est détecté dans un détecteur (8), son intensité étant déterminée en fonction de la longueur d'ondes et/ou de la durée.

16. Procédé selon l'une quelconque des revendications précédentes, caractérisé a) par l'exposition du tissu au rayonnement dans la plage de longueurs d'ondes de 720 - 800 nm, ce qui permet d'activer, dans le tissu, une fluorescence biphotonique, b) par la détection de l'intensité d'un premier signal fluorescent pour une longueur d'onde de 460 +/- 30 nm et d'un deuxième signal de fluorescence pour une longueur d'onde de 550 +/- 30 nm,

 a) par la détermination du rapport entre les intensités du premier et du deuxième signal fluorescent, et
 b) par la génération d'un signal en fonction du rapport déterminé à l'étape c).

17. Procédé selon l'une quelconque des revendications précédentes, **caractérisé en ce que**

**EP 1 889 039 B1**

a) le tissu est exposé au rayonnement dans la plage de longueurs d'ondes entre 500-550 nm, et

b) **en ce qu'**une première intensité d'une fluorescence biphotonique est détectée pour une longueur d'ondes de 340 +/- 40 nm,

c) **en ce que** le tissu est exposé à un rayonnement dans la plage de longueur d'ondes entre 720 - 800 run, et

d) **en ce qu'**une deuxième intensité d'une fluorescence biphotonique est détectée pour une longueur d'ondes de 460 +/- 40 nm,

e) **en ce que** le rapport entre la première intensité déterminée à l'étape b) et la deuxième intensité déterminée à l'étape d) est déterminé, et

f) **en ce qu'**un signal est généré en fonction du rapport déterminé à l'étape e).

18. Procédé selon l'une quelconque des revendications précédentes, **caractérisé en ce**

a) **que** le tissu est exposé à un rayonnement dans la plage de longueurs d'ondes entre 720 - 800 nm, et

b) en ce qu'une première intensité d'une fluorescence biphotonique est détectée pour une longueur d'ondes de 460 +/- 40 nm.

19. Procédé selon la revendication 18, **caractérisé en ce que** le rayonnement est reproduit dans le tissu par l'intermédiaire d'un objectif, et **en ce que** l'objectif est décalé progressivement le long du sens de propagation du rayonnement envoyé par la source de rayonnement, les étapes a) et b) étant effectuées pour plusieurs positions de l'objectif de sorte que le point d'activation est guidé dans le sens de propagation du rayonnement à travers le tissu et qu'un signal de fluorescence est déterminé pour plusieurs profondeurs de tissu.

20. Procédé selon la revendication 19, **caractérisé en ce que** le procédé est mis en oeuvre au niveau d'un tissu sain, et **en ce que** les intensités ainsi déterminées du signal de fluorescence sont mémorisées en tant que valeurs de référence.

21. Procédé selon la revendication 19 et 20, **caractérisé en ce que**

a) le procédé selon la revendication 19 est mis en oeuvre au niveau d'un tissu à caractériser,

b) **en ce que** les intensités déterminées pour chaque profondeur sont mises en rapport avec les intensités respectives du signal de référence mémorisé,

c) **en ce qu'**un signal est généré en fonction du rapport déterminé à l'étape b).

22. Procédé selon la revendication 21, **caractérisé en ce que** le signal est généré lorsque le rapport déterminé à l'étape b) s'écarte approximativement de 30 % de la valeur 1.

23. Procédé selon l'une quelconque des revendications précédentes, **caractérisé en ce**

a) **que** l'exposition du tissu à un rayonnement est effectuée à un faisceau de rayons laser à courte impulsion dans la zone de longueurs d'ondes de 720 - 880 nm ou de 980 - 1080 nm,

b) en ce que l'intensité du rayonnement rémittent est détecté pour la moitié de la longueur d'ondes du rayonnement utilisé selon l'étape a) aux fins de l'exposition du tissu à un rayonnement.

24. Procédé selon la revendication 23, **caractérisé en ce que** le rayonnement est reproduit dans le tissu par l'intermédiaire d'un objectif, et **en ce que** l'objectif est décalé progressivement le long du sens de propagation du rayonnement émis par la source de rayonnement, les étapes a) et b) étant effectuées pour plusieurs positions de l'objectif de sorte que le point de concentration est guidé dans le sens de propagation du rayonnement à travers le tissu et qu'un signal de fluorescence est déterminé pour plusieurs profondeurs de tissu.

25. Procédé selon la revendication 24, **caractérisé en ce que** le procédé est mis en oeuvre au niveau d'un tissu sain, et **en ce que** les intensités ainsi déterminées du signal de fluorescence sont mémorisées en tant que valeurs de référence.

26. Procédé selon la revendication 24 et 25, **caractérisé en ce**

a) **que** le procédé selon la revendication 23 est mis en oeuvre au niveau d'un tissu à caractériser,

b) en ce que les intensités déterminées pour chaque profondeur sont mises en rapport avec les intensités respectives du signal de référence,

c) en ce qu'un signal est généré en fonction du rapport déterminé à l'étape b).

**27.** Procédé selon la revendication 26, **caractérisé en ce que** le signal est généré lorsque le rapport déterminé à l'étape b) s'écarte approximativement de 40 % de la valeur 1.

**28.** Dispositif servant à la mettre en oeuvre un procédé selon l'une quelconque des revendications précédentes, comprenant :

- une source de rayonnement (2) servant à émettre un rayonnement orienté servant à exposer le tissu (1) à caractériser à un rayonnement, laquelle est configurée pour activer, dans le tissu, un renvoi de rayonnement caractéristique du tissu,
- le rayonnement ayant pénétré le tissu présentant, dans une plage d'activation s'étendant de manière transversale par rapport à son sens de propagation, une intensité suffisante pour activer un renvoi de rayonnement dans le tissu, et
- des moyens de mise en forme de rayonnement (3), qui sont configurés pour marquer sur le rayonnement émis par la source de rayonnement (2) un profil d'intensité tel que la zone d'activation couvre une pluralité de cellules du tissu et que le renvoi du rayonnement activé lors l'exposition du tissu au rayonnement est lié aux caractéristiques du tissu recouvrant les cellules,
- les moyens de mise en forme de rayonnement (3) étant réalisés de manière à répartir le rayonnement émis par la source de rayonnement en au moins deux faisceaux de rayons partiels (4a, 4b, 4c), la plage activation étant définie par la position des faisceaux de rayons partiels,
- des moyens de reproduction (5, 6) étant prévus, lesquels sont réalisés pour concentrer les faisceaux de rayons partiels (4a, 4b, 4c) respectivement dans le tissu (1) de telle manière que les points de concentration (51a, 51 b, 51c) pour les faisceaux de rayons partiels sont espacés les uns des autres, et
- un détecteur (8) servant à détecter le renvoi de rayonnement, qui génère un signal électrique en fonction du rayonnement détecté,

**caractérisé en ce**
**que** le détecteur (8) est configuré de manière à détecter le renvoi de rayonnement de manière globale à partir toutes les plages de concentration de tous les faisceaux de rayons partiels concentrés dans le tissu (1).

**29.** Dispositif selon la revendication 28, **caractérisé en ce que** les moyens de reproduction sont disposés, vu depuis la source de rayonnement, avant ou après les moyens de mise en forme du rayonnement.

**30.** Dispositif selon la revendication 28, **caractérisé en ce que** les moyens de reproduction comprennent plusieurs objectifs, et **en ce que** chaque faisceau de rayons partiel est concentré sur un objectif propre dans le tissu.

**31.** Dispositif selon l'une quelconque des revendications 28 à 30, **caractérisé en ce que** les moyens de reproduction (5, 6) comprennent au moins un objectif (5) servant à concentrer le rayonnement ainsi qu'une unité d'ajustement (6), à l'aide de laquelle l'au moins un objectif peut être déplacé le long du sens de propagation du rayonnement afin de déplacer le point de concentration de l'objectif le long dudit sens et pour permettre un balayage du tissu dans un plan, qui s'étend de manière perpendiculaire par rapport à la surface de tissu.

**32.** Dispositif selon la revendication 31, **caractérisé en ce que** l'unité d'ajustement (6) et les objectifs sont intégrés dans un boîtier, qui peut être posé sur le tissu à examiner et qui peut être déplacé par coulissement de manière parallèle par rapport à la surface du tissu.

**33.** Dispositif selon la revendication 32, **caractérisé par** des éléments de réglage à moteur électrique et/ou micromécaniques, à l'aide desquels le boîtier peut être déplacé par coulissement de manière parallèle par rapport à la surface du tissu.

**34.** Dispositif selon l'une quelconque des revendications 28 à 33, **caractérisé par** une fibre optique (30), qui rapproche le rayonnement généré par la source de rayonnement du tissu à caractériser.

**35.** Dispositif selon la revendication 34, **caractérisé en ce que** la fibre optique (30) présente les moyens de reproduction et/ou les moyens de mise en forme de rayonnement.

**36.** Dispositif selon la revendication 35, **caractérisé en ce que** les moyens de reproduction (5, 6) et/ou les moyens de mise en forme de rayonnement sont réalisés d'un seul tenant avec la fibre optique.

**37.** Dispositif selon l'une quelconque des revendications 28 à 36, **caractérisé par** une unité d'analyse servant à analyser le signal électrique généré par le détecteur (8).

**38.** Dispositif selon la revendication 37, **caractérisé en ce que** l'unité d'analyse convertit le signal électrique généré par le détecteur (8) en un signal d'information d'image.

**39.** Dispositif selon la revendication 38, **caractérisé en ce que** l'unité d'analyse présente un écran (40) servant à représenter visuellement le signal d'information d'image.

# FIG 1

# FIG 2

FIG 3

## FIG 4A

P

51

z

5

x

11

## FIG 4B

P

x

12

z

11a

13

FIG 5

EP 1 889 039 B1

FIG 6

EP 1 889 039 B1

FIG 7

EP 1 889 039 B1

FIG 8

# FIG 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5034613 A
- US 6166385 A
- US 2002122246 A1
- US 5413108 A
- WO 0007514 A
- DE 19927724 A1

**In der Beschreibung aufgeführte Nicht-Patentliteratur**

- **H. VAN DEN BERGH.** *Med. Laser Appl.,* 2003, vol. 18 (1), 20
- **W. DENK ; J.H. STRICKLER ; W.W. WEBB.** 2-Photon laser scanning fluorescence microscopy. *Science,* 1990, vol. 248, 73-76
- **ÜBERSICHT W.R. ZIPFEL et al.** *Nature Biotechnology,* 2003, vol. 21 (11), 1369-1377
- **K. KÖNIG et al.** *J. Biomed. Opt.,* 2003, vol. 8 (3), 432-439
- **W. R. ZIPFEL ; R. M. WILLIAMS ; W. W. WEBB.** *nature biotechnology,* November 2003, vol. 21-11
- **M. J. LEVENE et al.** In vivo multiphoton microscopy of deep brain tissue. *J Neurophysiol,* 2004, vol. 91, 1908-1912
- **P. THEER et al.** Two-Photon imaging to a depth of 1000 mm in living brain tissue... *Optics Lett.,* 2003, vol. 28 (12